# EUROPEAN PATENT APPLICATION

(11) **EP 4 123 026 A1**
(43) Date of publication of application: **25.01.2023**
(21) Application number: 21753938.6
(22) Date of filing: 14.02.2021
(51) Int. Cl.: C12N 15/63, A61K 38/02, A61K 48/00, A61P 35/00, A61P 35/02, C12Q 1/6886

(54) **NUCLEIC ACID CONSTRUCT, AND THERAPEUTIC OR DIAGNOSTIC AGENT FOR MISMATCH REPAIR DEFICIENT CANCERS COMPRISING NUCLEIC ACID CONSTRUCT**

(30) Priority: 14.02.2020 JP 2020023874
(71) Applicant: Public University Corporation Yokohama City University, Yokohama-shi, Kanagawa 236-0027 (JP)
(72) Inventor: ADACHI, Noritaka, Yokohama-shi, Kanagawa 236-0027 (JP)
(74) Representative: Mewburn Ellis LLP
(86) International application number: PCT/JP2021/005382
(87) International publication number: WO 2021/162121

(57) **Abstract**

Disclosed is means which enables simple and rapid detection of the presence or absence of mismatch repair activity, and which is useful for diagnosis and treatment of mismatch repair-deficient cancers. In an integrated-type nucleic acid construct provided by the present invention, [promoter region], [5'-side region + first homologous region], and [second homologous region + 3'-side region] are placed in the same nucleic acid molecule. In a divided-type nucleic acid construct, [promoter region], [5'-side region + first homologous region], and [second homologous region + 3'-side region] are placed in two different nucleic acid molecules. The nucleic acid construct of the present invention can be used as a therapeutic agent for mismatch repair-deficient cancer, as a diagnostic agent for mismatch repair-deficient cancer, or as a companion diagnostic agent for predicting an effect of an anticancer drug for mismatch repair-deficient cancer, containing the nucleic acid construct.

## Description

### TECHNICAL FIELD

The present invention relates to a nucleic acid construct, and a therapeutic agent or a diagnostic agent for mismatch repair-deficient cancer, comprising the nucleic acid construct.

### BACKGROUND ART

Mismatch repair is one of the DNA damage-repairing mechanisms cells possess, and plays an important role in maintenance of stability of the genome (Non-Patent Documents 1 and 2). In human cells, proteins encoded by genes such as *MSH2, MSH6, MLH1*, and *PMS2* are involved in mismatch repair, and cells deficient in these factors lose the mismatch repair activity. Abnormalities of factors that affect gene expression of these mismatch repair proteins also cause mismatch repair deficiency.

Mismatch repair deficiency causes various cancers (when mispairing (mismatch) of bases in DNA remains unrepaired, it leads to accumulation of genomic mutations, resulting in high carcinogenicity). A number of deficiencies of mismatch repair factors have been reported, and their examples include not only Lynch syndrome (also called hereditary non-polyposis colorectal cancer (HNPCC)), which is a well-known familial (genetic) tumor, but also various sporadic (non-genetic) cancers (especially colorectal cancer and endometrial cancer) (Non-Patent Document 3).

Diagnosis of a mismatch repair-deficient cancer is carried out based on microsatellite instability (MSI) or by immunostaining of the four factors described above. However, their results do not show a rate of concordance of 100% (Non-Patent Document 4). Further, in the latter method, mismatch repair deficiencies caused by abnormalities of factors other than the four factors may be missed (this applies also to genetic diagnosis on the four factors). Both methods require several days to several weeks for the diagnosis.

Regarding the treatment, immune checkpoint inhibitors (such as anti-PD-1 antibodies) have been found quite recently to produce a high response rate in mismatch repair-deficient cancers, and are therefore attracting a lot of attention (Non-Patent Documents 3 and 5). However, immune checkpoint inhibitors have side effects of autoimmune diseases due to immune suppression, and lethal cases have been reported. Thus, development of safer and less expensive therapeutic methods is also important.

### PRIOR ART DOCUMENT(S)

### NON-PATENT DOCUMENT(S)

Non-Patent Document 1: Jiricny J (2006) The multifaceted mismatch-repair system. Nat Rev Mol Cell Biol 7(5): 335-346.
Non-Patent Document 2: Harfe BD and Jinks-Robertson S (2000) DNA mismatch repair and genetic instability. Annu Rev Genet 34: 359-399.
Non-Patent Document 3: Le DT et al. Science. 2017 Jul 28; 357(6349): 409-413
Non-Patent Document 4: Hampel H et al. N Engl J Med 352: 1851-1860, 2015
Non-Patent Document 5: Le DT et al. N Engl J Med 2015; 372: 2509-2520.
Non-Patent Document 6: Stark JM et al. Mol Cell Biol. 2004 Nov; 24(21): 9305-9316.
Non-Patent Document 7: Mendez-Dorantes C et al. Genes Dev. 2018 Apr 1; 32(7-8): 524-536.
Non-Patent Document 8: Ochiai H et al. Genes Cells. 2010 Aug; 15(8): 875-885.

### SUMMARY OF THE INVENTION

### PROBLEMS TO BE SOLVED BY THE INVENTION

If there is a method that enables simple and rapid detection of the presence or absence of mismatch repair activity in cells, tissues, or individuals, the method is expected to be extremely useful for diagnosis and treatment of mismatch repair-deficient cancers including Lynch syndrome irrespective of the types of the cancers. For example, in cancer treatment using an immune checkpoint inhibitor, if whether mismatch repair is normal or not can be diagnosed in advance, the risk of occurrence of a serious side effect due to administration of the immune checkpoint inhibitor to a patient in whom it produces low response rate can be avoided.

Thus, an object of the present invention is to provide a means that enables simple and rapid detection of the presence or absence of mismatch repair activity, and is useful for diagnosis and treatment of mismatch repair-deficient cancers.

### MEANS FOR SOLVING THE PROBLEMS

As a result of intensive study, the present inventors discovered that, by dividing a protein-encoding gene sequence into two fragments, making the two fragments each have a region homologous to each other, designing a nucleic acid construct in which these fragments are incorporated in an expression vector, and then introducing the nucleic acid construct into a cell, the gene sequence is reconstructed by the single-strand annealing (SSA) mechanism in cells deficient in mismatch repair activity, leading to expression of the activity of the encoded protein, thereby completing the present invention.

More specifically, the present invention is an invention related to a nucleic acid construct comprising: a promoter region; and a 5'-side region and a 3'-side region of a gene sequence encoding a protein; the 5'-side region having a first homologous region linked downstream thereof, the 3'-side region having a second homologous region linked upstream thereof, the first homologous region and the second homologous region being homologous to each other, and use of the nucleic acid construct. The invention includes the following modes.

[1] A nucleic acid construct comprising: a promoter region; and a 5'-side region and a 3'-side region of a gene sequence encoding a protein; the 5'-side region having a first homologous region linked downstream thereof, the 3'-side region having a second homologous region linked upstream thereof, the first homologous region and the second homologous region being homologous to each other.
[2] The nucleic acid construct according to [1], wherein the nucleic acid construct is a circular nucleic acid construct comprising, downstream of the promoter region, the 5'-side region and the 3'-side region, or a linear nucleic acid construct prepared by cleaving the circular nucleic acid construct between the 5'-side region and the 3'-side region; or a linear nucleic acid construct comprising, downstream of the promoter region, the 5'-side region and the 3'-side region.
[3] A nucleic acid construct comprising: a promoter region; a 5'-side region and a 3'-side region of a gene sequence encoding a protein; a first homologous region linked downstream of the 5'-side region; and a second homologous region linked upstream of the 3'-side region; the regions being placed in two different nucleic acid molecules, wherein a first nucleic acid molecule comprises, downstream of the promoter region, the 5'-side region and the first homologous region, and a second nucleic acid molecule comprises the 3'-side region and the second homologous region.
[4] The nucleic acid construct according to any one of [1] to [3], wherein the 5'-side region and the 3'-side region are regions designed by dividing the gene sequence encoding the protein into two parts such that an overlapping region is included in each part, wherein the overlapping region present in the 3'-end portion within the 5'-side region is the first homologous region, and the overlapping region present in the 5'-end portion within the 3'-side region is the second homologous region.
[5] The nucleic acid construct according to any one of [1] to [3], wherein the first homologous region and the second homologous region are each composed of a non-coding sequence or at least part of a sequence encoding a protein different from the previously-mentioned protein.
[6] The nucleic acid construct according to any one of [1] to [5], comprising a poly-A addition signal downstream of the 3'-side region.
[7] The nucleic acid construct according to any one of [1] to [6], wherein the first homologous region and the second homologous region each have a chain length of 4 bases to 10,000 bases.
[8] The nucleic acid construct according to any one of [1] to [7], wherein the homology between the first homologous region and the second homologous region is 40% to 100%.
[9] The nucleic acid construct according to any one of [1] to [8], wherein the gene sequence is a gene sequence encoding a protein that acts to decrease cell survival rate, or a protein whose intracellular expression is detectable.
[10] The nucleic acid construct according to [9], wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.
[11] A therapeutic agent for mismatch repair-deficient cancer, the agent comprising the nucleic acid construct according to any one of [1] to [10], wherein the gene sequence is a gene sequence encoding a protein that acts to decrease cell survival rate.
[12] The therapeutic agent according to [11], wherein the gene sequence is a sequence of a suicide gene, a DNA damage-inducing gene, or a DNA repair-inhibiting gene.
[13] A diagnostic agent for mismatch repair-deficient cancer, the agent comprising the nucleic acid construct according to any one of [1] to [10].
[14] The diagnostic agent according to [13], wherein the gene sequence is a gene sequence encoding a protein whose intracellular expression is detectable.
[15] A companion diagnostic agent for predicting an effect of an anticancer drug for mismatch repair-deficient cancer, the agent comprising the nucleic acid construct according to any one of [1] to [10].
[16] The companion diagnostic agent according to [15], wherein the anticancer drug is an immune checkpoint inhibitor.
[17] The companion diagnostic agent according to [15] or [16], wherein the gene sequence is a gene sequence encoding a protein whose intracellular expression is detectable.
[18] A therapeutic method for mismatch repair-deficient cancer, the method comprising administering the nucleic acid construct according to any one of [1] to [10] to a patient with the mismatch repair-deficient cancer, wherein the gene sequence is a gene sequence encoding a protein that acts to decrease cell survival rate.
[19] A diagnostic method for mismatch repair-deficient cancer, the method comprising:
   introducing the nucleic acid construct according to any one of [1] to [10] into cancer cells of a cancer patient; and
   measuring expression of the protein.
[20] The method according to [19], wherein the expression of the protein is measured by directly or indirectly measuring the activity of the protein.
[21] The method according to [19] or [20], wherein the cancer cells are cells separated from the cancer patient, and the introduction of the nucleic acid construct into the cancer cells is carried out in vitro.
[22] The method according to [19] or [20], wherein:
   the protein is a protein whose intracellular expression is detectable as a signal;
   the introduction of the nucleic acid construct into the cancer cells is carried out by administering the nucleic acid construct to the cancer patient; and
   whether or not the signal of the protein is detected from a cancer lesion is investigated.
[23] The method according to [19] or [20], wherein:
   the protein is a secretory protein whose intracellular expression is detectable;
   the introduction of the nucleic acid construct into the cancer cells is carried out by administering the nucleic acid construct to the cancer patient; and
   the activity of the protein in blood separated from the patient after the administration of the nucleic acid construct is measured.
[24] A method of predicting an effect of an anticancer drug for a mismatch repair-deficient cancer, the method comprising:
   introducing the nucleic acid construct according to any one of [1] to [10] into cancer cells of a cancer patient; and
   measuring expression of the protein.
[25] The method according to [24], wherein the anticancer drug is an immune checkpoint inhibitor.
[26] The method according to [24] or [25], wherein the cancer cells are cells separated from the patient, and the introduction of the nucleic acid construct into the cancer cells is carried out in vitro.
[27] The method according to [24] or [25], wherein:
   the protein is a protein whose intracellular expression is detectable as a signal;
   the introduction of the nucleic acid construct into the cancer cells is carried out by administering the nucleic acid construct to the patient; and
   whether or not the signal of the protein is detected from a cancer lesion is investigated.
[28] The method according to [24] or [25], wherein:
   the protein is a secretory protein whose intracellular expression is detectable;
   the introduction of the nucleic acid construct into the cancer cells is carried out by administering the nucleic acid construct to the cancer patient; and
   the activity of the protein in blood separated from the patient after the administration of the nucleic acid construct is measured.
[29] A method of producing the nucleic acid construct according to [3], the method comprising:
   amplifying the first nucleic acid molecule by PCR using, as a template, an expression vector for the protein, the vector containing: the promoter region; the gene sequence encoding the protein; and a poly-A addition signal; and using set 1 of forward primer 1-1 that is set upstream of the promoter and reverse primer 1-2 that is set in a partial region α in the gene sequence; and
   amplifying the second nucleic acid molecule by PCR using the expression vector as a template, and using set 2 of forward primer 2-1 that is set in a partial region β which is another partial region in the gene sequence and is positioned upstream of the partial region α, and reverse primer 2-2 that is set downstream of the poly-A addition signal or between the gene sequence and the poly-A addition signal.

### EFFECT OF THE INVENTION

By the present invention, a nucleic acid construct with which mismatch repair deficiency can be detected utilizing single-strand annealing is provided. By the nucleic acid construct of the present invention, the presence or absence of mismatch repair activity can be detected in a transient expression system in a very short time. By utilizing a gene sequence encoding a protein that acts to decrease the cell survival rate, such as a suicide gene, mismatch repair-deficient cancer cells can be efficiently killed. Further, by utilizing a gene sequence encoding a protein whose intracellular expression is detectable, such as a luciferase gene or a fluorescent protein gene, a mismatch repair-deficient cancer can be detected. The present invention significantly contributes to diagnosis and treatment of various mismatch repair-deficient cancers including Lynch syndrome irrespective of the types of the cancers. Further, the nucleic acid construct of the present invention is also applicable to search for factors that positively or negatively regulate, and inhibitors or activators of, mismatch repair or single-strand annealing reaction, and to prediction of whether or not a gene mutation identified in a cancer patient is a mutation that deteriorates the mismatch repair activity.

### BRIEF DESCRIPTION OF THE DRAWINGS

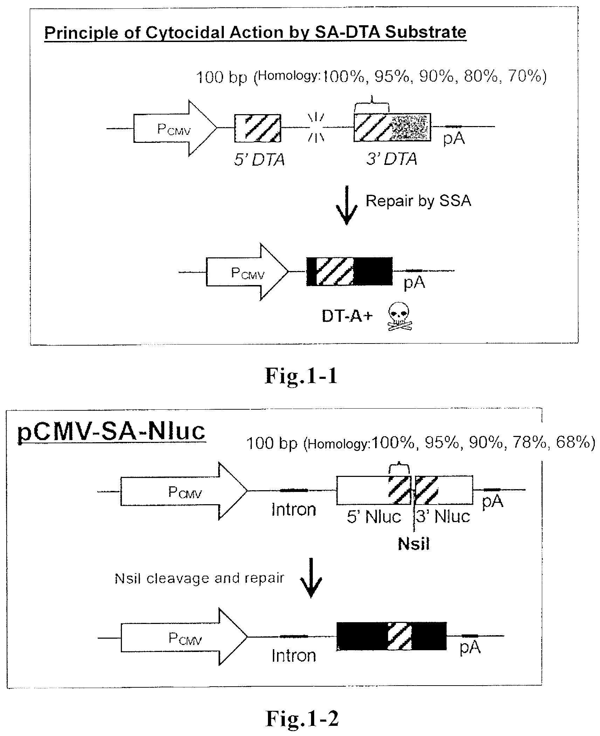

Fig. 1-1 is a diagram illustrating the structure and the mechanism of the nucleic acid construct (integrated type) utilizing the suicide gene *DT-A,* prepared in Example 1-1.
Fig. 1-2 is a diagram illustrating the structure and the mechanism of the nucleic acid construct (integrated type) utilizing a luciferase gene, prepared in Example 1-1.
Fig. 2 is a structural drawing of pIRES used for the preparation of plasmid-vector-type nucleic acid constructs in Examples (the structural drawing in pIRES Vector Information of Clontech was partially modified).
Fig. 3 is a structural drawing of an integrated-type nucleic acid construct utilizing the suicide gene *DT-A,* prepared in Examples, wherein the homology of the homologous regions (overlapping region) was set to 100%.
Fig. 4 is a structural drawing of an integrated-type nucleic acid construct utilizing the suicide gene *DT-A,* prepared in Examples, wherein the homology of the homologous regions (overlapping region) was decreased.
Fig. 5 is a structural drawing of an integrated-type nucleic acid construct utilizing a luciferase gene, prepared in Examples.
Fig. 6 shows the cytocidal effects of integrated-type nucleic acid constructs prepared using the suicide gene *DT-A* (SA-DTA construct), investigated by the colony formation method.
Fig. 7 shows the cytocidal effects of integrated-type SA-DTA constructs investigated by a growth inhibition test.
Fig. 8 shows the cytocidal effects of integrated-type SA-DTA constructs investigated by a growth inhibition test.
Fig. 9 shows the result of a luciferase assay performed in Nalm-6 cells transfected with an integrated-type nucleic acid construct (SA-Nluc construct) prepared using a luciferase gene.
Fig. 10 shows the result of a luciferase assay performed in Nalm-6 cells transfected with an integrated-type SA-Nluc construct.
Fig. 11 shows an example of the result of an experiment using integrated-type SA-Nluc constructs having varied homologies between the homologous regions.
Fig. 12 shows the result of investigation of the influence of the difference in the size of the homologous region in an integrated-type SA-DTA construct on the cytocidal effect.
Fig. 13 shows an example of the result of a luciferase assay performed in Nalm-6 cells or *MSH2* revertant cells thereof (Nalm-6 + *MSH2*) transfected with integrated-type pCMV-SA-Nluc-spacer constructs.
Fig. 14 shows an example of the result of a luciferase assay performed in HCT116 cells or *MLH1* revertant cells thereof (HCT116 +*MLH1*) transfected with integrated-type pCMV-SA-Nluc-spacer constructs.
Fig. 15 shows an example of the result of a luciferase assay performed in Nalm-6 cells transfected with an SA-Nluc construct of an integrated-type (pCMV-SA-Nluc) or a divided-type (pCMV-5'Nluc + 3'Nluc-pA).
Fig. 16 shows an example of the result of a luciferase assay performed in Nalm-6 cells transfected with divided-type SA-Nluc constructs having homologous regions with decreased homologies.
Fig. 17 shows an example of the result of evaluation of the cytocidal effect in Nalm-6 cells transfected with an SA-DTA construct of an integrated type (pCMV-SA-DTA) or a divided type (pCMV-5'DTA + 3'DTA-pA), which evaluation was carried out by the colony formation method.
Fig. 18 is a structural drawing of an integrated-type pCMV-SA-TK construct utilizing the suicide gene *HSV-TK*, prepared in Examples.
Fig. 19 shows an example of the result of evaluation of the cytocidal effect in Nalm-6 cells transfected with an integrated-type pCMV-SA-TK construct, which evaluation was carried out by a growth inhibition test. The survival rate of the cells 6 days after the gene transfection is expressed as a relative value with respect to the rate observed without addition of ganciclovir (GANC), which is taken as 100.
Fig. 20 is an example of the result of evaluation of the cytocidal effect in Nalm-6 cells transfected with an integrated-type pCMV-SA-TK construct, which evaluation was carried out by the colony formation method.
Fig. 21 is an example of the result of a luciferase assay carried out using a culture supernatant of cells transfected with an integrated-type pCMV-SA-SecNluc construct that utilizes a secretory luciferase gene.
Fig. 22 is an example of the result of a luciferase assay performed in Nalm-6 cells transfected with an SA-Nluc construct whose homologous regions have a chain length of 40 bp and a decreased homology therebetween (100%, 90%, 70%, 60%, or 50% homology).

### MODE FOR CARRYING OUT THE INVENTION

The nucleic acid construct of the present invention comprises: a promoter region; and a 5'-side region and a 3'-side region of a gene sequence encoding a protein. A first homologous region is linked downstream of the 5'-side region, and a second homologous region is linked upstream of the 3'-side region. The base sequences of the first homologous region and the second homologous region are homologous to each other.

The nucleic acid construct of the present invention includes an integrated-type nucleic acid construct that is composed of a single nucleic acid molecule, and a divided-type nucleic acid construct composed of two nucleic acid molecules.

In the integrated-type nucleic acid construct, [promoter region], [5'-side region + first homologous region], and [second homologous region + 3'-side region] are placed in the same nucleic acid molecule. A poly-A addition signal may or may not be placed downstream of the 3'-side region. Since mRNAs that appropriately function without a poly-A, such as a histone mRNA, are known, the poly-A addition signal may be omitted also in the nucleic acid construct of the present invention.

In the divided-type nucleic acid construct, [promoter region], [5'-side region + first homologous region], and [second homologous region + 3'-side region] are placed in two different nucleic acid molecules. The first nucleic acid molecule comprises [promoter region] and [5'-side region + first homologous region], wherein the 5'-side region + the first homologous region are placed downstream of the promoter region. The second nucleic acid molecule comprises [second homologous region + 3'-side region]. A poly-A addition signal may or may not be placed downstream of [second homologous region + 3'-side region]. Similarly to the integrated-type construct, the poly-A addition signal may be omitted.

When the term "nucleic acid construct of the present invention" is simply used in the present description, it includes both the integrated type and the divided type.

In the nucleic acid construct of the present invention, the promoter is not limited, and may be any promoter as long as a promoter activity can be exerted in a cell (typically a mammalian cell such as a human cell). In general, a promoter that constitutively exerts a strong promoter activity in the cell into which the nucleic acid construct of the present invention is introduced may be preferably used. Or, an inducible promoter that exerts the promoter activity under certain conditions may be used. In the following Examples, a human cytomegalovirus promoter that constitutively exerts a strong promoter activity in a cell was used. However, the promoter is not limited thereto. Further, the promoter is not limited to a virus-derived promoter, and the origin of the promoter is not limited as long as it is a sequence having a promoter activity in a cell, preferably in a human cell.

The gene sequence employed for the nucleic acid construct of the present invention is not limited as long as it is a gene sequence encoding a protein. In the present description, the protein encoded by this gene sequence may be referred to as protein A. Protein A is preferably a protein whose expression level can be rapidly and simply measured. For example, the protein used as protein A is preferably a protein whose activity can be measured directly, or indirectly using, for example, a phenotypic change that occurs in the cells due to the activity of the protein, rather than a protein requiring measurement of the amount of production or accumulation of the protein itself. Examples of such a protein include proteins whose intracellular expression can be detected based on a signal such as luminescence due to reaction with a substrate, or fluorescence of the protein itself. Another example is a protein that acts to decrease the cell survival rate. In this case, the activity of the protein can be indirectly measured as a decrease in the cell survival rate.

The term "measurement of expression (the expression level) of protein A" includes measurement of the amount of production or accumulation of protein A, and measurement of the activity of protein A. As described above, in the present invention, it is preferred to measure the expression of protein A by directly or indirectly measuring the activity of protein A. In other words, it is preferred to employ, as protein A, a protein whose activity can be directly or indirectly measured.

Specific examples of the gene encoding a protein that acts to decrease the cell survival rate include, but are not limited to, suicide genes, DNA damage-inducing genes, and DNA repair-inhibiting genes.

Examples of the suicide genes include genes encoding a protein which by itself is toxic or injurious to cells, genes encoding a protein which acts on another compound to produce a toxic substance, and genes encoding a naturally occurring or non-naturally occurring protein that inhibits an endogenous protein to exert cytotoxicity by the dominant negative effect. Specific examples of the suicide genes include the diphtheria toxin A fragment gene (*DT-A*) (the DT-A protein itself has toxicity to cells, and kills the cells), the herpesvirus-derived thymidine kinase gene (*HSV-tk*) (HSV-tk protein acts on ganciclovir, 5-iodo-2'-fluoro-2'deoxy-1-beta-D-arabino-furanosyl-uracil (FIAU), or the like to convert it to a toxic substance having a DNA synthesis-inhibiting activity; use of this gene as a suicide gene requires treatment of the cells with ganciclovir, FIAU, or the like), and the p53 gene (its overexpression induces cell cycle arrest or apoptosis to cause cell death). Further specific examples of the suicide genes include naturally-occurring nucleases represented by restriction enzymes and meganucleases; and artificial nucleases represented by ZFN and TALEN, and the CRISPR system (these are also included in specific examples of the DNA damage-inducing genes). However, the suicide genes are not limited to these specific examples.

Examples of the gene encoding a protein whose intracellular expression is detectable include, but are not limited to, luminescent enzyme genes such as luciferase genes (including genes encoding a secretory luciferase); fluorescent protein genes (genes encoding a naturally occurring or an artificial fluorescent protein including GFP, CFP, OFP, RFP, and YFP, and modified types thereof); cell surface antigen genes; secretory protein genes; and membrane protein genes. Luminescent enzyme genes and fluorescent protein genes are genes encoding a protein whose intracellular expression can be detected as a signal such as luminescence or fluorescence, and such genes are included in examples of genes that may be preferably used in the present invention.

The nucleic acid construct of the present invention can be largely divided into two modes based on the type of the first and second homologous regions irrespective of whether the nucleic acid construct is an integrated type or a divided type.

In the first mode, the first and second homologous regions are derived from part of the gene sequence from which the 5'-side region and the 3'-side region are derived. Thus, each of the first and second homologous regions has part of the gene sequence encoding protein A, or a base sequence having not less than a certain level of homology thereto. In this mode, the 5'-side region and the 3'-side region can be designed by dividing the gene sequence encoding the protein into two parts such that an overlapping region is included in each part. The overlapping region present in the 3'-end portion in the 5'-side region functions as the first homologous region, and the overlapping region present in the 5'-end portion in the 3'-side region functions as the second homologous region. In the following Examples, A-1-1 to A-1-3, B-1-1 to B-1-4, and C-1 are nucleic acid constructs of this first mode.

In the second mode, the first and second homologous regions are derived from a base sequence that is different from the gene sequence from which the 5'-side region and the 3'-side region are derived. The homologous regions are each composed of a non-coding sequence or at least part of a sequence encoding a protein different from protein A. In the latter case, the protein different from protein A may be a protein that is not naturally occurring in the cells to be transfected with the nucleic acid construct or in the organism species to which the nucleic acid construct is to be administered. Or, the protein may be a protein naturally occurring in the organism species. The non-coding sequence may be an artificial sequence that is not naturally occurring.

In either mode, the homology between the first homologous region and the second homologous region is not limited as long as the homology is sufficient for allowing single-strand annealing between the homologous regions. As described in the following Examples, the nucleic acid construct of the present invention functions even in cases where the size of each homologous region is reduced to 20 bp, and where the homology is decreased to 85% at the same time. Therefore, the nucleic acid construct functions as long as each homologous region contains a continuous region of at least 20 bp having a homology of not less than 85%, for example, not less than 90%, not less than 95%, or 100%. Thus, the homology value is not limited at all. The homology between the homologous regions when the entire homologous regions are compared is not limited, and may be, for example, 40% to 100%, 45% to 100%, 51% to 100%, 52% to 100%, 53% to 100%, 54% to 100%, 55% to 100%, 56% to 100%, 57% to 100%, 58% to 100%, 59% to 100%, 60% to 100%, 65% to 100%, 70% to 100%, 75% to 100%, 80% to 100%, 85% to 100%, 90% to 100%, or 95% to 100%. Regarding the upper limit value, the homology may be less than 100%, for example, not more than 98%, not more than 97%, not more than 96%, or not more than 95%. The size of each homologous region is not limited as long as the chain length is sufficient for allowing single-strand annealing in the cells. The size may be, for example, not less than 4 bases, not less than 10 bases, or not less than 15 bases. The upper limit of the size is also not limited. The size is usually not more than 10,000 bases, and may be, for example, not more than 5000 bases, not more than 1000 bases, not more than 700 bases, not more than 500 bases, or not more than 200 bases. In the present invention, the chain length of a nucleic acid is expressed using the unit "bases". The "bases" means "base pairs" in cases where the nucleic acid is a double-stranded nucleic acid.

The chain length of the protein A gene sequence employed for the 5'-side region needs to be set such that a protein A fragment capable of exerting the activity of protein A is not expressed from the 5'-side region when single-strand annealing does not occur. The size of the 5'-side region may be appropriately set depending on the type of protein A. In general, the size of the protein A fragment employed for the 5'-side region may be not more than 35/100, for example, not more than 30/100, not more than 25/100, or not more than 20/100 of the entire length of protein A.

The 5'-side region and the 3'-side region used for the nucleic acid construct of the present invention can be prepared by PCR amplification using appropriate primers, from a known plasmid in which a gene encoding protein A is incorporated, or from cultured cells or a cDNA library of an organism species that expresses protein A. To the 3'-end of each of the two gene fragments encoding protein A, a stop codon is introduced. In the PCR amplification, in addition to the stop codon, an appropriate adaptor sequence used for cloning of the nucleic acid construct of the present invention and/or the like may be introduced to each of the two gene fragments. Usually, a poly-A addition signal is placed downstream of the 3'-side region. Usually, in a divided-type nucleic acid construct, a poly-A addition signal is placed in the second nucleic acid molecule. However, as described above, the poly-A addition signal is not an indispensable element.

The nucleic acid construct of the present invention may be a construct in which the elements such as the 5'-side region are incorporated in a plasmid vector(s), may be a construct in which the elements such as the 5'-side region are incorporated in a virus vector(s), or may be in the form of a nucleic acid fragment(s) in which the elements are not incorporated in a vector. In the case of a divided-type nucleic acid construct, the construct may be in a form in which both of the two nucleic acid molecules are incorporated in vectors, may be in the form of nucleic acid fragments in which neither of the nucleic acid molecules is incorporated in a vector, or may be in a form in which one of the nucleic acid molecules is incorporated in a vector while the other is not incorporated in a vector.

In the integrated-type nucleic acid construct, [promoter region] - [5'-side region + first homologous region] - [second homologous region + 3'-side region] are placed in this order from the 5'-side toward the downstream side in a vector. In cases where a poly-A addition signal is included, it is usually placed downstream of the 3'-side region. Also in cases where the construct is in the form of a nucleic acid fragment not incorporated in a vector, the arrangement of the elements from the 5'-side is the same as described above. When an integrated-type nucleic acid construct using a plasmid vector is used in applications such as a therapeutic agent or diagnostic agent, the construct may be used as it is in the form of a circular nucleic acid construct containing [5'-side region + first homologous region] and [second homologous region + 3'-side region] in this order downstream of the promoter region, or may be used in a linear form prepared by cleaving this circular nucleic acid construct between [5'-side region + first homologous region] and [second homologous region + 3'-side region]. A version 1 nucleic acid construct using a virus vector may be a linear nucleic acid construct containing [5'-side region + first homologous region] and [second homologous region + 3'-side region] in this order downstream of the promoter region. The integrated-type nucleic acid construct not incorporated in a vector may be a linear nucleic acid construct containing [5'-side region + first homologous region] and [second homologous region + 3'-side region] in this order downstream of the promoter region.

The arrangement of the elements in the divided-type nucleic acid construct is as described above. In the first nucleic acid molecule, the promoter region and the 5'-side region + the first homologous region may be placed from the 5'-side toward the downstream side. In the second nucleic acid molecule, the second homologous region + the 3'-side region may be included, and a poly-A addition signal may be placed downstream thereof. In cases where the first nucleic acid molecule is circular, it may be used in the form of the circular molecule itself in various applications, or may be used in the form of a linear molecule prepared by cleaving the circular molecule at an arbitrary position in the downstream side of the first homologous region (the position may be several hundred bases to about 2000 or more bases distant from the 3'-end of the first homologous region). In cases where the second nucleic acid molecule is circular, it may be used in the form of the circular molecule itself in various applications, or may be used in the form of a linear molecule prepared by cleaving the circular molecule at an arbitrary position in the upstream side of the second homologous region (the position may be several hundred bases to about 2000 or more bases distant from the 5'-end of the second homologous region).

The nucleic acid construct of the present invention may be either DNA or RNA. In general, DNA is preferred in cases of a plasmid-vector-type nucleic acid construct. Typical examples of the virus-vector-type nucleic acid construct include a nucleic acid construct that is a double-stranded DNA in the form of a virus expression plasmid. Examples of the virus-vector-type nucleic acid construct also include a virus expressed from a virus expression plasmid. In cases where the vector is an RNA virus vector, the nucleic acid construct that is a virus expressed from the virus expression plasmid is RNA. In cases of a DNA virus vector, this nucleic acid construct is DNA. In cases where the nucleic acid construct is in a form in which it is not incorporated in a vector, the nucleic acid construct is usually DNA. The nucleic acid construct of the present invention may comprise a nucleotide analog in part thereof. Examples of the nucleotide analog include, but are not limited to, bridged nucleic acids such as LNA, ENA, and PNA; and modified bases such as Super T (registered trademark) and Super G (registered trademark).

Examples of a simple production method for the divided-type nucleic acid construct include a method in which the two nucleic acid molecules are amplified using, as a template, an ordinary protein A expression vector containing a promoter, a gene sequence encoding protein A, and a poly-A addition signal in this order from the upstream side, and using primers arranged at appropriate positions. As the primers, set 1 of forward primer 1-1 that is set upstream of the promoter and reverse primer 1-2 that is set in an appropriate partial region α in the gene sequence, and set 2 of forward primer 2-1 that is set in a partial region β which is another partial region in the gene sequence and which is positioned upstream of the partial region α, and a reverse primer 2-2 that is set downstream of the poly-A addition signal or between the gene sequence and the poly-A addition signal, may be used. The first nucleic acid molecule can be amplified by PCR using the primer set 1, and the second nucleic acid molecule can be amplified by PCR using the primer set 2. Regarding the primer set 2, in cases where a primer that is set downstream of the poly-A addition signal is used as the reverse primer 2-2, a second nucleic acid molecule containing the poly-A addition signal can be obtained. In cases where a primer that is set between the gene sequence and the poly-A addition signal is used as the reverse primer 2-2, a second nucleic acid molecule containing no poly-A addition signal can be obtained. Based on how the regions to be set as the partial region α and the partial region β are selected, the size of the homologous region can be controlled. Further, by introducing a mutation(s) to at least one of the reverse primer 1-2 and the forward primer 2-1, nucleic acid molecules whose homologous regions have a decreased homology can be easily prepared.

In the integrated-type nucleic acid construct, an appropriate spacer sequence may be placed between the 3'-end of the first homologous region and the 5'-end of the second homologous region. In cases of the divided-type nucleic acid construct, in the first nucleic acid molecule, a first spacer sequence may be linked to the 3'-end of the first homologous region, and further, in the second nucleic acid molecule, a second spacer sequence may be linked to the 5'-end of the second homologous region. Each spacer sequence may contain an appropriate restriction site for cleavage of the nucleic acid construct, or a sequence for in vivo cleavage. The length and the base sequence of each spacer sequence are not limited. The length and the base sequence of the spacer sequence are not at all limited, and spacers having various lengths and sequences may be used. The length of the spacer sequence may be, for example, not less than 100 bases, not less than 500 bases, not less than 1000 bases, not less than 1500 bases, not less than 2000 bases, not less than 2500 bases, not less than 3000 bases, not less than 3500 bases, or not less than 4000 bases. Although there is no upper limit of the length, the length is usually not more than 10,000 bases.

The nucleic acid construct of the present invention can be preferably used as a therapeutic agent or diagnostic agent for mismatch repair-deficient cancer, or as a companion diagnostic agent for predicting an effect of an anticancer drug for mismatch repair-deficient cancer.

Mismatch repair-deficient cancers have been reported in various cancers. Specific examples of the mismatch repair-deficient cancers that have been reported so far include colorectal cancer, endometrial cancer, gastric cancer, esophagus cancer, ovarian cancer, breast cancer, pancreatic cancer, hepatobiliary cancer, urinary tract cancer, small intestinal cancer, prostate cancer, and liver cancer (for example, Non-Patent Document 3). It is expected that mismatch repair-deficient cancers will be found also in various cancers other than these (including cancers of various animal species including non-human animals) in the future. It has also been reported that the mismatch repair activity is decreased by molecular targeted cancer therapies using an EGFR inhibitor, a BRAF inhibitor or the like (Science 20 Dec 2019: Vol. 366, Issue 6472, pp 1473-1480, DOI:10.1126/science.aav4474). Another well-known example of a mismatch repair-deficient cancer is Lynch syndrome, which is a familial (genetic) cancer (another name: hereditary non-polyposis colorectal cancer (HNPCC)). Examples of the mismatch repair-deficient cancer to be targeted when the nucleic acid construct of the present invention is used as a therapeutic agent or diagnostic agent include various cancers including the specific examples described above. When the term "colorectal cancer" is simply used in the present invention, it includes not only nonhereditary colorectal cancer, but also Lynch syndrome (hereditary non-polyposis colorectal cancer).

In cases where the nucleic acid construct of the present invention is used as a therapeutic agent for mismatch repair-deficient cancer, the gene sequence encoding protein A may be a gene sequence encoding a protein that acts to decrease the cell survival rate. In cases of a cancer which is not deficient in mismatch repair, single-strand annealing of the nucleic acid construct is blocked by the mismatch repair mechanism, so that the gene sequence encoding protein A is not reconstructed in the cells, and protein A is not expressed. In cases of a cancer which is deficient in mismatch repair, single-strand annealing of the nucleic acid construct occurs in the cells to reconstruct the gene sequence encoding protein A in the cells, so that protein A is expressed to enable efficient killing of the cancer cells. In cases of a cancer in which mismatch repair is not deficient, the effect to kill the cancer cells cannot be obtained. Therefore, before the administration of the therapeutic agent of the present invention, whether or not the cancer is a mismatch repair-deficient cancer may be investigated using, for example, the diagnostic agent of the present invention, and the therapeutic agent of the present invention may be administered to the patient when the patient is diagnosed with mismatch repair-deficient cancer. In cases where protein A is a protein that acts on another compound to produce a toxic substance, the compound that is the target of the action is also administered to the patient. For example, in cases where protein A is HSV-tk, the nucleic acid construct of the present invention may be administered in combination with ganciclovir, FIAU, or the like.

In cases where the nucleic acid construct of the present invention is used as a diagnostic agent for mismatch repair-deficient cancer, the nucleic acid construct of the present invention is introduced into cancer cells of the cancer patient, and expression of protein A is measured. The measurement of the expression of protein A is preferably carried out by directly or indirectly measuring the activity of protein A as described above. As the gene sequence encoding protein A employed for the 5'-side region and the 3'-side region, a gene sequence encoding a protein whose intracellular expression is detectable may be preferably used, and a gene sequence encoding a protein that acts to decrease the cell survival rate may also be used. In cases where mismatch repair is not deficient in the cell, single-strand annealing of the nucleic acid construct is blocked by the mismatch repair mechanism, so that the gene sequence encoding protein A is not reconstructed in the cell, and the activity of protein A is not detected. In cases where mismatch repair is deficient in the cell, single-strand annealing of the nucleic acid construct occurs to reconstruct the gene sequence encoding protein A in the cell, so that the protein A activity is detected.

In one mode of the diagnostic agent for mismatch repair-deficient cancer, the cancer cells are cells separated from a cancer patient, and the introduction of the nucleic acid construct into the cancer cells is carried out in vitro. Among the modes of use described later, (1) and (3) are specific examples of this mode. In this mode, although either a protein whose intracellular expression is detectable or a protein that acts to decrease the cell survival rate may be used as protein A, the former is more preferred. In this mode, the cancer patient includes solid cancer patients and blood cancer patients. Cells in a cancer tissue separated from a cancer patient by biopsy or surgery, or, in cases of leukemia, cancer cells in blood collected from a cancer patient, may be used. If desired, the cancer cells may be concentrated before the introduction of the nucleic acid construct. The introduction of the nucleic acid construct may be transient.

After the introduction of the nucleic acid construct, expression of protein A, preferably the activity of protein A, in the cancer cells is measured. In cases where protein A is a protein whose intracellular expression is detectable, the expression (activity) of protein A can be measured by measuring a signal of protein A. In cases where protein A is a protein that acts to decrease the cell survival rate, the expression (activity) of protein A can be measured by measuring the survival rate of the cancer cells after the introduction of the nucleic acid construct. Preferably, in the measurement of the expression of protein A in cancer cells, cells which have normal mismatch repair activity or which are not deficient in mismatch repair activity are used as an MMR-proficient control, and, after introducing the nucleic acid construct into the cancer cells and the control cells, the expression of protein A is compared between the cancer cells and the control cells.

Preferred examples of the control cells include non-cancerous cells (cells not deficient in the mismatch repair activity) collected from the same cancer patient. Or, a known cell line not deficient in the mismatch repair activity may be used as the control cells. A number of wild-type mammalian cell lines having no mutation in MMR-related genes (such as *MSH2*, *MSH6, MLH1*, *PMS2*, *EPCAA1*, *MSH3, PMS1*, *MLH3, ARIDIA*, *SETD2, EXO1*, *RPA1*, *RPA2, RPA3, POLD*, *LIG1*, and *LIG3*) are known, and any of such known lines may be used as the MMR-proficient control cells. Specific examples of known mammalian cell lines having normal mismatch repair activity include, but are not limited to, human cell lines such as HT1080 (a human fibrosarcoma cell line), U2OS (a human osteosarcoma-derived cell line), HeLa (a human cervical cancer-derived cell line), MCF-7 (a human breast adenocarcinoma-derived cell line), HAP1 (a human chronic myelogenous leukemia-derived cell line), HEK293 (a human embryonic kidney-derived cell line), TIG-7, TIG-3 (a human lung-derived cell line), iPS cells (human induced pluripotent stem cells; established from normal human cells), and ES cells (human embryonic stem cells).

Further, if desired, cells deficient in the mismatch repair activity may be used as an additional control. Examples of such MMR-deficient control cells include cell lines derived from mismatch repair-deficient cancers. Various cell lines derived from mismatch repair-deficient cancers having a mutation(s) in the MMR-related genes described above are known, and any of such cells may be used as the MMR-deficient control cells. Or, a cell line prepared by introducing a mutation(s) to one or more MMR-related genes can also be used. Since the technique for gene knockout has been established, MMR-deficient cells of various animal species can be prepared by knocking out one or more MMR-related genes in mammalian cultured cells having normal mismatch repair activity. Specific examples of known MMR-deficient human cell lines include Nalm-6 (a human B-cell leukemia-derived cell line), LoVo (a human colon adenocarcinoma-derived cell line), Jurkat (a human T-cell leukemia-derived cell line), and C-33 A (a human cervical cancer-derived cell line), which are deficient in *MSH2*; HCT116 (a human colon adenocarcinoma-derived cell line), SK-OV-3 (a human ovarian cancer-derived cell line), and DU-145 (a human prostate cancer-derived cell line), which are deficient in *MLH1*; DLD-1(a human colon adenocarcinoma-derived cell line), which is deficient in *MSH6;* and MMR-deficient human cell lines available from Horizon Discovery (the following gene-deficient cells are available as genetically modified HAP1 cell lines (human chronic myelogenous leukemia-derived cell lines): an MSH2-deficient line, an *MLH7-deficient* line, an *MSH6*-deficient line, and a *PMS2*-deficient line). Specific examples of the non-human animal cell lines include, but are not limited to, MMR-deficient mice (frozen embryos) available from The NCI Mouse Repository *(Msh2-*deficient mice, *Mlh1-*deficient mice, and *Msh6*-deficient mice); the canine cancer cell line TYLER2 (Dodd G. Sledge et al., Proceedings of the 103rd Annual Meeting of the American Association for Cancer Research; 2012 Mar 31-Apr 4; Chicago, IL. Philadelphia (PA): AACR; Cancer Res 2012; 72(8 Suppl): Abstract nr 5258. doi:1538-7445. AM2012-5258); and the MMR-deficient canine cell Angus *(MSH6-*deficient) (Sunetra Das et al., Mol Cancer Ther. Author manuscript; available in PMC 2020 Feb 1. Published in final edited form as: Mol Cancer Ther. 2019 Aug; 18(8): 1460-1471. Published online 2019 Jun 7. doi: 10.1158/1535-7163. MCT-18-1346).

When the expression level or activity level of protein A in the cancer cells is higher than the expression level or activity level of protein A in the MMR-proficient control cells, the cancer in the cancer patient is indicated to be a mismatch repair-deficient cancer. In cases where protein A is a protein whose intracellular expression is detectable as a signal, when the detected signal of protein A is higher in the cancer cells than in the MMR-proficient control cells, the cancer in the cancer patient is indicated to be a mismatch repair-deficient cancer. In cases where protein A is a protein that acts to decrease the cell survival rate, when the survival rate of the cancer cells is lower than the survival rate of the MMR-proficient control cells, the cancer in the cancer patient is indicated to be a mismatch repair-deficient cancer.

When the expression level or activity level of protein A in the cancer cells is not higher than (about the same as, or lower than) the expression level or activity level of protein A in the MMR-proficient control cells, the cancer in the cancer patient is indicated not to be a mismatch repair-deficient cancer. In cases where protein A is a protein whose intracellular expression is detectable as a signal, when the level of the signal detected in the cancer cells is about the same as or lower than the level in the MMR-proficient control cells, the cancer in the cancer patient is indicated not to be a mismatch repair-deficient cancer. In cases where protein A is a protein that acts to decrease the cell survival rate, when the survival rate of the cancer cells is about the same as or higher than the survival rate of the MMR-proficient control cells, the cancer in the cancer patient is indicated not to be a mismatch repair-deficient cancer.

In another mode of the diagnostic agent for mismatch repair-deficient cancer, protein A is a protein whose intracellular expression is detectable as a signal; the introduction of the nucleic acid construct into the cancer cells is carried out by administering the nucleic acid construct to the patient; and whether or not the signal of protein A is detected from a cancer lesion is investigated. Among the modes of use described later, (2) is a specific example of this mode. The nucleic acid construct may be topically administered into the tumor or into the vicinity of the tumor of the cancer patient, or may be systemically administered. Topical administration is more preferred. In this mode, the cancer patient is typically a solid cancer patient. From the viewpoint of detecting the signal of protein A from the cancer lesion in the body of the cancer patient, protein A is preferably a protein that generates the signal without a reaction with a substrate, for example, a fluorescent protein. When the signal of protein A is detected from the cancer lesion (for example, when the signal from the cancer lesion is clearly higher than the signal from a non-cancerous site in the vicinity of the tumor), the cancer of the cancer patient is indicated to be a mismatch repair-deficient cancer. When no signal of protein A is detected from the cancer lesion, or when a signal as low as that of a non-cancerous site in the vicinity of the tumor is detected, a possibility that the cancer of the cancer patient is not a mismatch repair-deficient cancer is indicated. If desired, cancer cells may be collected from the patient, and the nucleic acid construct may be introduced thereto in vitro to further confirm whether or not the cancer is a mismatch repair-deficient cancer.

In still another mode, protein A is a secretory protein whose intracellular expression is detectable; the introduction of the nucleic acid construct into the cancer cells is carried out by administering the nucleic acid construct to the cancer patient; and the activity of protein A in blood separated from the patient after the administration of the nucleic acid construct is measured. Among the modes of use described later, (4) is a specific example of this mode. The nucleic acid construct may be topically administered into the tumor or into the vicinity of the tumor of the cancer patient, or may be systemically administered. Topical administration is more preferred. In this mode, the cancer patient is typically a solid cancer patient. In cases where the cancer of the patient is deficient in mismatch repair, secretory protein A is expressed from the protein A gene reconstructed in the cancer cells, and secreted to the outside of the cancer cells. Therefore, the activity of protein A can be detected using a blood sample of the cancer patient. Since the activity of protein A secreted into the blood is detected in vitro, a protein that generates a signal by reaction with a substrate can also be preferably used. Typical examples of such a protein include secretory luminescent enzymes, especially secretory luciferases. As the gene sequence encoding the secretory protein such as a secretory luciferase, a known secretory protein coding sequence may be used as it is, or the gene sequence may be prepared by adding a sequence encoding a secretory signal to a protein coding sequence. As a negative control sample, for example, a blood sample collected from a patient before the administration of the nucleic acid construct, or a culture supernatant obtained by introducing the nucleic acid construct to an animal cell line not deficient in mismatch repair and culturing the resulting cells may be used. As a positive control, for example, a culture supernatant obtained by introducing the nucleic acid construct into a cell line deficient in the mismatch repair activity (such as a cell line derived from a mismatch repair-deficient cancer) and culturing the resulting cells may be used. When the signal of protein A is detected in a blood sample (for example, when a signal of protein A higher than that of a negative control sample is detected in a blood sample), the cancer of the patient is indicated to be a mismatch repair-deficient cancer. When the signal of protein A is not detected in a blood sample, in particular, when a signal higher than that of a negative control is not detected (in other words, when the level of the signal detected is about the same as or lower than the level in a negative control sample), a possibility that the cancer of the cancer patient is not a mismatch repair-deficient cancer is indicated. If desired, cancer cells may be collected from the patient, and the nucleic acid construct may be introduced thereto to further confirm whether or not the cancer is a mismatch repair-deficient cancer.

In cases where a patient is diagnosed with mismatch repair-deficient cancer by the diagnostic technique for mismatch repair-deficient cancer according to the present invention, an immune checkpoint inhibitor is likely to be effective for the patient, so that an immune checkpoint inhibitor may be preferably administered to the patient. In cases where a patient is diagnosed as not having mismatch repair-deficient cancer, severe side effects are likely to occur without production of a sufficient cancer therapeutic effect by the immune checkpoint inhibitor, so that an anticancer drug other than an immune checkpoint inhibitor may be preferably administered to the patient.

Examples of modes of the diagnostic agent of the present invention include, but are not limited to, the following modes.

(1) Cancer cells collected from a cancer patient are cultured, and the nucleic acid construct of the present invention is introduced into the cultured cancer cells, followed by investigating whether or not a signal of protein A is detected (or investigating whether or not a cytocidal effect by protein A is found). If desired, non-cancerous cells (such as peripheral blood cells) collected from the same cancer patient may be used as a control. In cases where protein A is a luciferase, a substrate of the luciferase is added for the detection reaction. In cases where protein A is a fluorescent protein, a fluorescence signal may be detected using a luminometer or the like. When the signal of protein A is detected (in particular, when a signal of protein A higher than that of control non-cancerous cells is detected), the cancer of the patient can be judged to be deficient in mismatch repair. When no signal of protein A is detected, or when a signal as low as that of control non-cancerous cells is detected, the cancer of the patient can be judged not to be deficient in mismatch repair.
   The method of measuring the cell survival rate is well known in the art, and the measurement can be easily carried out using a commercially available kit or the like. When cancer cells into which the nucleic acid construct has been introduced show a decreased survival rate (in particular, when the survival rate is lower than that of control non-cancerous cells), the cancer of the patient can be judged to be deficient in mismatch repair. When no decrease in the survival rate of cancer cells is found, or when the survival rate is almost the same as that of control non-cancerous cells, the cancer of the patient can be judged not to be deficient in mismatch repair.
(2) The nucleic acid construct of the present invention is administered to a patient, and whether or not a signal of protein A is detected from the cancer lesion is investigated. When the signal is detected, the cancer of the patient can be judged to be deficient in mismatch repair. When no signal is detected, it can be judged that the cancer of the patient may not be deficient in mismatch repair. In this mode, a protein whose intracellular expression is detectable is used as protein A. Biotoxicity of the protein employed needs to be sufficiently low.
(3) Cancer cells present in blood are isolated from a cancer patient, and then concentrated when necessary, followed by introducing the nucleic acid construct of the present invention to the cells and investigating whether or not a signal of protein A is detected (or whether or not a cytocidal effect (a decreased cell survival rate) due to protein A is found) (in cases of leukemia).
(4) A nucleic acid construct that employs a gene sequence encoding a secretory luciferase as the gene sequence encoding protein A is administered to a patient. Thereafter, a blood sample is collected, and the presence or absence of luciferase reaction is investigated using the blood sample. In cases where the cancer of the patient is deficient in mismatch repair, the secretory luciferase is expressed from the luciferase gene reconstructed in the cancer cells, and secreted to the outside of the cells. Thus, the luciferase reaction can be detected using the blood sample. In cases where the cancer of the patient is not deficient in mismatch repair, the secretory luciferase is not produced, so that the luciferase reaction is not detected in the blood sample.

The diagnostic agent of the present invention is also useful as a companion diagnostic agent for predicting an effect of an anticancer drug for mismatch repair-deficient cancer. Examples of the anticancer drug include immune checkpoint inhibitors. Specific examples of the immune checkpoint inhibitors include, but are not limited to, anti-PD-1 antibodies such as nivolumab, pembrolizumab, spartalizumab, and cemiplimab; anti-PD-L1 antibodies such as avelumab, atezolizumab, and durvalumab; and anti-CTLA-4 antibodies such as ipilimumab and tremelimumab. Specific examples of modes of use as a companion diagnostic agent include those described above for the diagnostic agent. In other words, also in cases where the nucleic acid construct of the present invention is used as a companion diagnostic agent to predict an effect of an anticancer drug against mismatch repair-deficient cancer, similarly to the above-described diagnostic technique for mismatch repair-deficient cancer, the nucleic acid construct of the present invention may be introduced into cancer cells of a cancer patient, and expression of protein A (preferably the activity of protein A) may be measured to diagnose whether or not the patient has mismatch repair-deficient cancer. When the patient is diagnosed with mismatch repair-deficient cancer, the patient can be judged to be a patient in whom a desired anticancer effect is likely to be obtained with an anticancer drug against mismatch repair-deficient cancer, such as an immune checkpoint inhibitor (in other words, it can be predicted that an anticancer drug against mismatch repair-deficient cancer, such as an immune checkpoint inhibitor, is effective for the patient). Thus, to such a cancer patient, an immune checkpoint inhibitor may be preferably administered for the treatment of the cancer. It has been reported that immune checkpoint inhibitors may cause severe side effects. By preliminarily investigating whether or not the cancer is mismatch repair-deficient cancer, administration of an immune checkpoint inhibitor can be avoided in patients in whom it may produce side effects without producing a therapeutic effect.

Further, the nucleic acid construct of the present invention can also be applied to search for factors that positively or negatively regulate, and inhibitors or activators of, the reaction of mismatch repair or single-strand annealing, and to prediction of whether or not a gene mutation identified in a cancer patient is a pathological mutation that deteriorates the mismatch repair activity. By using a nucleic acid construct having a homology of 100% between the homologous regions as a control, and using a nucleic acid construct having a low homology in combination therewith, a drug or a mutation that affects the mismatch repair activity can be identified. For example, prediction of a pathogenic mutation may be carried out as follows. When a mutation is identified in gene X (gene X is preferably an MMR-related gene) in cancer cells of a cancer patient, an expression vector that expresses a mutant gene X having the same mutation and an expression vector that expresses wild-type gene X having no mutation are constructed, and these expression vectors are introduced into cells deficient in gene X (the cells may be cells prepared by knocking out gene X, or, if there is a known cell line completely deficient in the function of gene X, such a known cell line may be used). Further, nucleic acid constructs of the present invention (the construct having a homology of 100% and the low-homology construct) are introduced into the cells, and the expression level of protein A in the cells is measured. By comparing expression of protein A between the cells into which the nucleic acid construct having a homology of 100% is introduced and the cells into which the low-homology nucleic acid construct is introduced, and between the cells into which the mutant gene X is introduced and the cells into which wild-type gene X is introduced, whether or not the mutation is a pathological mutation that deteriorates the mismatch repair activity can be predicted.

In cases where the agent of the present invention is administered to a patient, the administration route may be either oral administration or parenteral administration. In general, parenteral administration such as intravenous administration, intraarterial administration, subcutaneous administration, or intramuscular administration is preferred. The administration may be systemic administration, administration into the tumor or to the vicinity of the tumor, or administration to a regional lymph node of the tumor. The dose is not limited, and may be about 1 pg to 10 g, for example, about 0.01 mg to 100 mg, in terms of the amount of the nucleic acid construct per day for a patient. The agent may be administered in a single dose or several divided doses per day. The agent may be administered every day, or may be administered every several days or several weeks.

The dosage form of the agent of the present invention to be administered to the patient is not limited. Depending on the administration route, the agent may be prepared by appropriately mixing the nucleic acid construct of the present invention with an additive(s) such as a pharmaceutically acceptable carrier, diluent, or excipient. Examples of the formulation include parenteral formulations such as drops, injection solutions, suppositories, and inhalants; and oral formulations such as tablets, capsules, granules, powders, and syrups. Preparation methods and additives that can be used are well known in the field of pharmaceutical formulations, and any of the methods and additives may be used.

In cases of a diagnostic agent to be used in vitro, the amount of the agent used for the treatment of the cells may be, for example, about 10 pg to 1 mg, e.g. about 0.1 ng to 100 µg, in terms of the amount of the nucleic acid construct per 1,000,000 cells. The dosage form may be a liquid agent supplemented, if desired, with an additive(s) or the like useful for stability or the like of the nucleic acid construct, or may be a powder agent prepared by freeze-drying of the nucleic acid construct.

In cases where the nucleic acid construct of the present invention is used as a therapeutic agent or a diagnostic agent, examples of the target cancer patient include cancer patients of various mammals such as humans, dogs, cats, ferrets, hamsters, mice, rats, horses, and pigs. The nucleic acid construct of the present invention is applicable to various animal species without changing the components except that the origin of the promoter is changed when necessary.

### EXAMPLES

The present invention is described below more concretely by way of Examples. However, the present invention is not limited to the following Examples.

### A. Experiment 1

### A-1. Method of Preparation of Vectors (DNA Constructs)

A construct (Figs. 1-1 and 1-2) which comprised a gene divided into two portions, each of which portions was given a homologous region, and expressed the function of the gene when the gene was reconstructed by single-strand annealing (SSA) was constructed as follows.

### A-1-1. Preparation of Construct with 100-bp Overlapping Region

### A-1-1-1. Construction of pCMV-SA-DTA Construct (100% Homology between Homologous Regions; 100-bp Overlap)

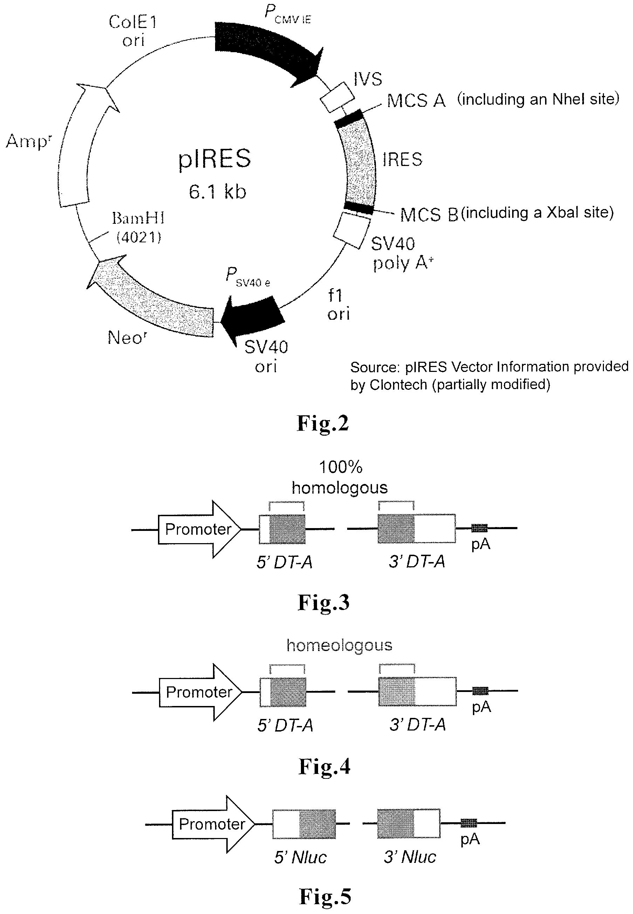

First, by PCR using pMC1DT-ApA (KURABO, Osaka, Japan) as a template, a 132-bp *5'DT-A* fragment (the region corresponding to positions 1 to 132 of the base sequence of the *DT-A* gene ORF shown in SEQ ID NO:9; SEQ ID NOs:11 and 12) and a 556-bp *3'DT A* fragment (the region corresponding to positions 33 to 588 of the base sequence of the *DT-A* gene ORF shown in SEQ ID NO:9; SEQ ID NOs:13 and 14) were obtained as two incomplete *DT-A* gene fragments. As a polymerase for the PCR, PrimeSTAR HS (trade name) DNA Polymerase (Takara Bio Inc., Otsu, Japan) was used. The primers used are shown below in Table 1. For amplification of the *5'DT-A* fragment, DTA-Fw (SEQ ID NO:1) and SSA-5'DTA-Rv (SEQ ID NO:2) were used to amplify a DNA fragment having a structure in which TGA TAGGGATAACAGGGTAATGC (stop codon + I-SceI recognition site + GC; SEQ ID NO:15) is linked to the 3'-end of the 132-bp 5*'DT-A* fragment, and in which sequences for In-Fusion reaction are added to both ends. For amplification of the *3'DT-A* fragment, SSA-3'DTA-Fw (SEQ ID NO:3) and DTA-Rv (SEQ ID NO:4) were used to amplify a DNA fragment having a structure in which a stop codon is linked to the 3'-end of the 556-bp *3'DT-A* fragment, and in which sequences for In-Fusion reaction are added to both ends. Subsequently, pIRES (Takara Bio Inc., Fig. 2) was digested with Nhel and XbaI to remove the IRES region and recover a fragment containing the CMV promoter and a poly-A addition signal. Using an In-Fusion HD Cloning Kit (Takara Bio Inc.), the 5'*DT-A* fragment and the 3'*DT-A* fragment were inserted downstream of the CMV promoter (the site where the IRES region had been present), to obtain the plasmid construct pCMV-SA-DTA (100% homology), which contained a structure in which [CMV promoter] - [5*'DT-A* fragment] - [3'*DT-A* fragment] - [poly-A addition signal] were linked together. The prepared plasmid was purified using a Qiagen Plasmid Plus Midi Kit (Qiagen K. K., Tokyo, Japan), and linearized by cleavage with I-SceI (New England Biolabs, Ipswich, MA, USA) before using the plasmid in transfection. Fig. 3 shows the I-Scel cleavage site in pCMV-SA-DTA. The cleavage occurs between *[5'DT-A* fragment] and [3'*DT-A* fragment]. The base sequence of the [5'*DT-A* fragment] - [3'*DT-A* fragment] portion is shown in SEQ ID NO:16.

### A-1-1-2. Construction of pCMV-SA-DTA Construct (100-bp Overlap) Having Decreased Homology between Homologous Regions

For preparation of pCMV-SA-DTA in which the homology between the homologous regions was decreased, a *3'DT-A* DNA fragment in which silent mutations were introduced in the DNA sequence (100 bp) homologous to *5'DT-A* was prepared by artificial gene synthesis (using the artificial synthetic gene service by GenScript; the DNA fragment delivered was in a state of being inserted in a vector). The silent mutations were introduced such that the homology to 5'*DT-A* was 95% (5 sites; SEQ ID NO:17), 90% (10 sites; SEQ ID NO:18), 80% (20 sites; SEQ ID NO:19), or 70% (30 sites; SEQ ID NO:20). Each of the four 3'*DT-A* fragments having different mutations prepared by the artificial gene synthesis was digested with PflMI to cut out and recover the fragment containing the mutated 3'*DT-A* from the vector. Subsequently, pCMV-SA-DTA (100% homology) was digested with PflMI to cleave it at positions before and after the *3'DT A* fragment, to thereby remove the *3'DT A* fragment having a homology of 100% and recover the fragment containing the CMV promoter, the *5'DT-A* fragment, and the poly-A addition signal. Using a DNA Ligation Kit <Mighty Mix> (Takara Bio Inc.), each 3'*DT-A* fragment having mutations was inserted downstream of the *5'DT-A* fragment (the site where the 3'*DT-*A fragment having a homology of 100% had been present) in the recovered fragment, to obtain pCMV-SA-DTA95 (95% homology), pCMV-SA-DTA90 (90% homology), pCMV-SA-DTA80 (80% homology), and pCMV-SA-DTA70 (70% homology), in each of which [CMV promoter] - [5'*DT-A* fragment] - [each *3'DT-A* fragment having mutations] - [poly-A addition signal] were linked together. These mutant constructs are also cleaved between *[5'DT-A* fragment] and [each *3'DT-A* fragment having mutations] by I-SceI digestion (Fig. 4).

### A-1-1-3. Construction of pCMV-SA-Nluc Construct (100% Homology between Homologous Regions; 99-bp Overlap)

Two *Nluc* gene fragments to be used for pCMV-SA-Nluc (100% homology), that is, a 5'-side gene fragment containing a 345-bp 5'*Nluc* fragment (the region corresponding to positions 1 to 345 of the base sequence of the *Nluc* gene shown in SEQ ID NO:21; SEQ ID NOs:23 and 24) and a 3'-side gene fragment containing a 267-bp *3'Nluc* fragment (the region corresponding to positions 247 to 513 of the base sequence of the *Nluc* gene shown in SEQ ID NO:21; SEQ ID NOs:25 and 26) were obtained by PCR amplification using the pNL1.1 [Nluc] Vector (Promega, Madison, WI, USA) as a template. As a polymerase for the PCR, PrimeSTAR HS (trade name) DNA Polymerase (Takara Bio Inc.) was used. The primers used are shown below in Table 1. For amplification of the *5'Nluc* fragment, Nluc-Fw (SEQ ID NO:5) and SSA-5'Nluc-Rv (SEQ ID NO:6) were used to amplify a 5'-side fragment having a structure in which a stop codon was linked to the 3'-end of the 345-bp *5'Nluc* fragment, and in which sequences for In-Fusion reaction were added to both ends. For amplification of the 3'*Nluc* fragment, SSA-3'Nluc-Fw (SEQ ID NO:7) and Nluc-Rv (SEQ ID NO:8) were used to amplify a 3'-side fragment having a structure in which sequences for In-Fusion reaction were added to both ends of the 267-bp *3'Nluc* fragment (including the stop codon taa; chain length, 270 bp). Subsequently, pIRES (Takara Bio Inc., Fig. 2) was digested with NheI and XbaI to remove the IRES region and recover a fragment containing the CMV promoter and the poly-A addition signal. Using an In-Fusion HD Cloning Kit (Takara Bio Inc.), the *5'Nluc* fragment and the *3'Nluc* fragment were inserted downstream of the CMV promoter (the site where the IRES region had been present) in the recovered fragment, to obtain the plasmid construct pCMV-SA-Nluc (100% homology), which contained a structure in which [CMV promoter] - [5'*Nluc* fragment] - [3'*Nluc* fragment] - [poly-A addition signal] were linked together. The prepared plasmid was purified using a Qiagen Plasmid Plus Midi Kit (Qiagen K. K.), and linearized by cleavage with NsiI (Takara Bio Inc.) before using the plasmid in transfection. Fig. 1-2 shows the NsiI cleavage site in pCMV-SA-Nluc. The cleavage occurs between [5*'Nluc* fragment] and [3'*Nluc* fragment]. The base sequence of the [5'*Nluc* fragment] - [3'*Nluc* fragment] portion is shown in SEQ ID NO:27.

### A-1-1-4. Construction of pCMV-SA-Nluc Construct (100-bp Overlap) Having Decreased Homology between Homologous Regions

For preparation of pCMV-SA-Nluc in which the homology between the homologous regions was decreased, a *3'Nluc* DNA fragment in which silent mutations were introduced in the DNA sequence (100 bp) homologous to *5'Nluc* was prepared by artificial gene synthesis (using the artificial synthetic gene service by GenScript; the DNA fragment delivered was in a state of being inserted in a vector). The silent mutations were introduced such that the homology to *5'Nluc* was 95% (5 sites; SEQ ID NO:28), 90% (10 sites; SEQ ID NO:29), 78% (22 sites; SEQ ID NO:30), or 68% (32 sites; SEQ ID NO:31). Each of the four 3'*Nluc* fragments having different mutations prepared by the artificial gene synthesis was digested with NsiI and PpuMI to cut out and recover the fragment containing the mutated 3'*Nluc* from the vector. Subsequently, pCMV-SA-Nluc (100% homology) was digested with NsiI and PpuMI to cleave it at positions before and after the *3'Nluc* fragment, to thereby remove the 3'*Nluc* fragment having a homology of 100% and recover the fragment containing the CMV promoter, the 5'*Nluc* fragment, and the poly-A addition signal. Using a DNA Ligation Kit <Mighty Mix> (Takara Bio Inc.), each *3'Nluc* fragment having mutations was inserted downstream of the 5'*Nluc* fragment (the site where the 3'*Nluc* fragment having a 100% homology had been present) in the recovered fragment, to obtain pCMV-SA-Nluc95 (95% homology), pCMV-SA-Nluc90 (90% homology), pCMV-SA-Nluc78 (78% homology), and pCMV-SA-Nluc68 (68% homology), in each of which [CMV promoter] - [5'*Nluc* fragment] - [each *3'Nluc* fragment having mutations] - [poly-A addition signal] were linked together. These mutant construct are also cleaved between [5'*Nluc* fragment] and [each 3'*Nluc* fragment having mutations] by Nsil digestion.

**[Table 1]**

| Primer name | Sequence (5'-3') | SEQ ID NO |
|---|---|---|
| DTA-Fw | ACTCACTATAGGCTAGCGTCCTCGCCATGGATCCTGATGATGTTG | 1 |
| SSA-5'DTA-Rv | CCGCCCCGACTCTAGAATgcattaccctgttatccctatcaTTGTGTACCAGATTTT GGC | 2 |
| SSA-3'DTA-Fw | TAGGGATAACAGGGTAATGCATCTTTTGTGATGGAAAACTTTTCTTCG | 3 |
| DTA-Rv | CCGCCCCGACTCTAGAA**TCA**CAAAGATCGCCTGACACGATTTCCTG | 4 |
| Nluc-Fw | TAATACGACTCACTATAGGCTAG | 5 |
| SSA-5'Nluc-Rv | GGCCGCCCCGACTCTAGAA**TCA**CGGCCGTCCGAAATAGTCGATC | 6 |
| SSA-3'Nluc-Fw | CGGCCGTGATTCTAGATGCATACCCTGTGGATGATCATCAC | 7 |
| Nluc-Rv | CCGCCCCGACTCTAGAGTCGCGG | 8 |

| | | |
|---|---|---|
| Single-underline: Restriction enzyme recognition site (GCTAGC, Nhel; TCTAGA, Xbal; attaccctgttatcccta, I-Scel) Double-underline: A site that anneals to the target gene sequence (The targets of Nluc-Fw and Nluc-Rv are vector sequences upstream and downstream, respectively, of the *Nluc* gene in pNL1.1 [Nluc] Vector.) Bold: Sequence for introduction of a stop codon (TGA) Lower-case letter portion of SSA-5'DTA-Rv: A sequence for introduction of stop codon + I-Scel recognition site + GC (SEQ ID NO:15) | | |

### A-1-2. Preparation of Construct with 40-bp Overlapping Region

Using the suicide gene *DT-A*, a construct in which an overlapping region was shortened to 40 bp was prepared.

### A-1-2-1. Construction of pCMV-SA-DTA40 Construct (100% Homology between Homologous Regions; 40-bp Overlap)

As a 3'*DT-A* fragment with a 40-bp overlap, the region corresponding to positions 93 to 588 of the *DT-A* gene ORF sequence shown in SEQ ID NO:9 (496 bp; SEQ ID NO:32) was employed. PCR was carried out using SSA-3'DTA-40bp-100-Fw (TAGGGATAACAGGGTAATGCATTCAAAAAGGTATACAAAAGCC; SEQ ID NO:38) and DTA-Rv (SEQ ID NO:4) as primers, and using pMC1DT-ApA (KURABO, Osaka, Japan) as a template, to amplify a DNA fragment having a structure in which sequences for In-Fusion reaction and the like were added to the 496-bp 3'*DT-A* fragment. pCMV-SA-DTA (100% homology) was digested with NsiI and Xbal to cleave it at positions before and after the *3'DT A* fragment, to thereby remove the *3'DT A* fragment having a homology of 100% and recover the fragment containing the CMV promoter, the 5'*DT-A* fragment, and the poly-A addition signal. Using an In-Fusion HD Cloning Kit (Takara Bio Inc.), the *3'DT A* fragment was inserted downstream of the 5'*DT-A* fragment (the site where the 3'*DT-*A fragment having a homology of 100% had been present) in the recovered fragment, to obtain the plasmid construct pCMV-SA-DTA40, which contained a structure in which [CMV promoter] - *[5'DT-A* fragment] - [40-bp-overlapping 3'*DT-A* fragment] - [poly-A addition signal] were linked together.

### A-1-2-2. Construction of pCMV-SA-DTA40-92.5 Construct (92.5% Homology between Homologous Regions; 40-bp Overlap)

A 3'*DT-A* fragment whose homology to the homologous region of the 5'*DT-A* fragment was 92.5% (SEQ ID NO:33) was obtained by PCR amplification using pMC1DT-ApA (KURABO, Osaka, Japan) as a template. PCR was carried out using SSA-3'DTA-40bp-92.5-Fw (TAGGGATAACAGGGTAATGCATTCAAAAAGGAATACAAAAACCAAAATCA GGTACAC; SEQ ID NO:39) and DTA-Rv (SEQ ID NO:4) as primers, and using pMC1DT-ApA (KURABO, Osaka, Japan) as a template, to amplify a DNA fragment having a structure in which sequences for In-Fusion reaction and the like were added to a 496-bp 3'*DT-A* fragment which had silent mutations at three sites in the 40-bp overlapping region. By the same procedure as in 1-2-1, the plasmid construct pCMV-SA-DTA40-92.5, which contained a structure in which [CMV promoter] - [5*'DT-A* fragment] - [40-bp-overlapping *3'DT-A* (92.5% homology) fragment] - [poly-A addition signal] were linked together, was obtained.

### A-1-3. Preparation of Construct with 20-bp Overlapping Region

Using the suicide gene *DT-A*, a construct in which an overlapping region was shortened to 20 bp was prepared.

### A-1-3-1. Construction of pCMV-SA-DTA20 Construct (100% Homology between Homologous Regions; 20-bp Overlap)

As a *3'DT A* fragment with a 20-bp overlap, the region corresponding to positions 113 to 588 of the *DT-A* gene ORF sequence shown in SEQ ID NO:9 (476 bp; SEQ ID NO:34) was employed. PCR was carried out using SSA-3'DTA-20bp-100-Fw (TAGGGATAACAGGGTAATGCATAGCCAAAATCTGGTACACAAGGA; SEQ ID NO:40) and DTA-Rv (SEQ ID NO:4) as primers, and using pMC1DT-ApA (KURABO, Osaka, Japan) as a template, to amplify a DNA fragment having a structure in which sequences for In-Fusion reaction and the like were added to the 476-bp *3'DT-A* fragment. By the same procedure as in 1-2-1, the plasmid construct pCMV-SA-DTA20, which contained a structure in which [CMV promoter] - [5'*DT-A* fragment] - [20-bp-overlapping *3'DT-A* fragment] - [poly-A addition signal] were linked together, was obtained.

### A-1-3-2. Construction of pCMV-SA-DTA20-95, pCMV-SA-DTA20-90, and pCMV-SA-DTA20-85 Constructs (95%, 90%, or 85% Homology between Homologous Regions; 20-bp Overlap)

*3'DT-A* fragments in each of which a silent mutation(s) was/were introduced to one site, two sites, or three sites in the 20-bp overlapping region such that the homology to the homologous region of the 5'*DT-A* fragment was decreased to 95%, 90%, or 85%, respectively (SEQ ID NOs: 35, 36, and 37), were amplified by PCR using pMC1DT-ApA (KURABO, Osaka, Japan) as a template. As primers, SSA-3'DTA-20bp-95-Fw (TAGGGATAACAGGGTAATGCATAGCCAAAATCAGGTACACAAGGA; SEQ ID NO:41) and DTA-Rv (SEQ ID NO:4) were used for the amplification of the *3'DT-*A fragment having a homology of 95%; SSA-3'DTA-20bp-90-Fw (TAGGGATAACAGGGTAATGCATAGCCAAAGTCAGGTACACAAGGA, SEQ ID NO:42) and DTA-Rv (SEQ ID NO:4) were used for the amplification of the 3'*DT-*A fragment having a homology of 90%; and SSA-3'DTA-20bp-85-Fw (TAGGGATAACAGGGTAATGCATAGCCAAAGTCAGGCACACAAGGA; SEQ ID NO:43) and DTA-Rv (SEQ ID NO:4) were used for the amplification of the *3'DT-*A fragment having a homology of 85%. By the same procedure as in 1-2-1, the plasmid constructs pCMV-SA-DTA20-95, pCMV-SA-DTA20-90, and pCMV-SA-DTA20-85, each of which contained a structure in which [CMV promoter] - [5'*DT-A* fragment] - [20-bp-overlapping 3'*DT-A* (95%, 90%, or 85% homology) fragment] - [poly-A addition signal] were linked together, were obtained.

### A-2. Evaluation of Cytocidal Effect in Human Cells, and Method of Luciferase Assay

The human pre-B cell line Nalm-6 (cancer cells deficient in the mismatch repair mechanism) and cells derived therefrom were cultured at 37°C in a 5% CO₂ incubator (So et al. (2004) Genetic interactions between BLM and DNA ligase IV in human cells. J. Biol. Chem. 279: 55433-55442).

*Msh2* revertant cells (*Msh2*+ cells) were obtained by knocking in *MSH2* cDNA into the human pre-B cell line Nalm-6 (cancer cells deficient in the mismatch repair mechanism; *Msh2-).* More specifically, a method in a past report (Restoration of mismatch repair functions in human cell line Nalm-6, which has high efficiency for gene targeting. Suzuki T, Ukai A, Honma M, Adachi N, Nohmi T. PLoS One. 2013 Apr 15;8(4):e61189. doi: 10.1371/journal.pone.0061189.) was employed. *Msh2*+ cells have the mismatch repair mechanism due to recovery by knock-in of *MSH2.* A construct showing a large difference in the luciferase activity or the cytocidal effect (that is, the recombination efficiency) between *Msh2-* cells and *Msh2*+ cells can be evaluated as a construct that responds sharply to the presence or absence of mismatch repair.

Gene transfer into the Nalm-6 cells and the cells derived therefrom was carried out by the electroporation method according to a past report (Saito S, Maeda R, Adachi N. Nat Commun. 2017 Jul 11;8:16112. doi:10.1038/ncomms16112.). After transfecting 2×10⁶ cells with each construct (1 µg), the cytocidal effect was investigated by one of the following methods.

### (1) Growth Inhibition Test

The cells after the gene transfer were plated on a 24-well dish at 1×10⁵ cells/ml, and the survival rate of the cells was measured at 24-hour intervals using CellTiter-Glo (CellTiter-Glo (registered trademark) Luminescent Cell Viability Assay, Promega).

### (2) Colony Formation Method

Twenty-four hours after the gene transfer, 200 or 5000 cells were plated on a 60-mm dish, and colonies were allowed to form for 17 days, followed by counting the number of colonies.

A luciferase assay was carried out as follows. With each construct (1 µg), 2×10⁶ Nalm-6 cells were transfected. The cells after the gene transfer were plated on a 12-well dish at 5×10⁵ cells/ml, and the luciferase activity (RLU value) was measured over time using the Nano-Glo Luciferase Assay System (Promega).

### A-3. Results

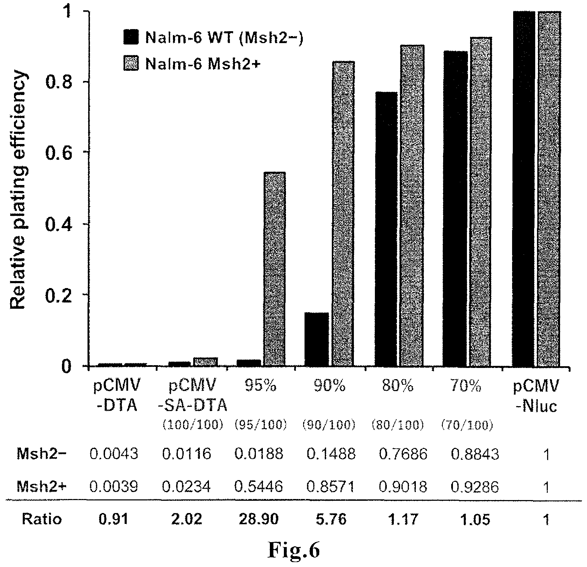

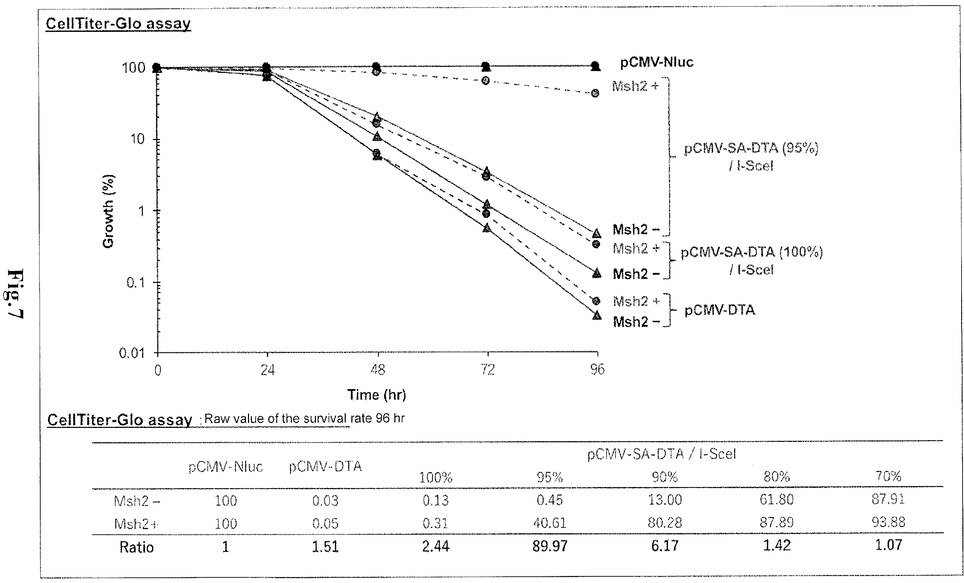

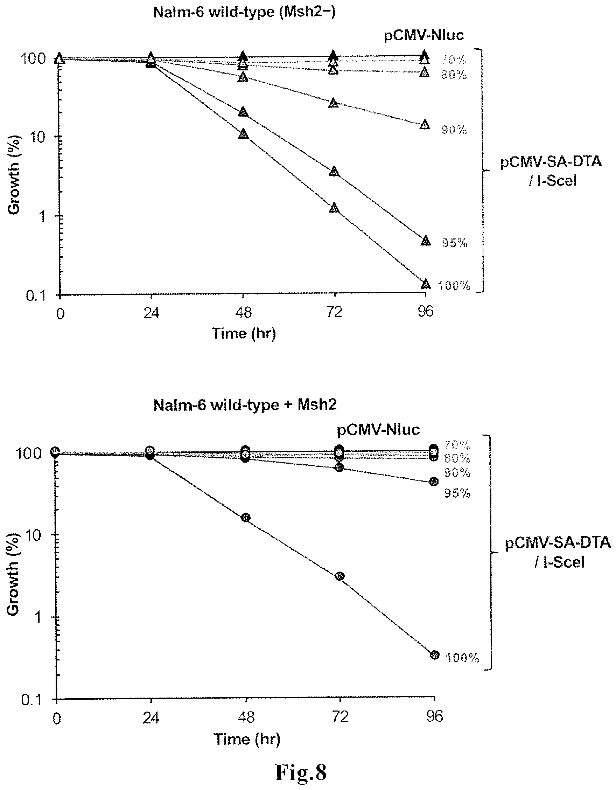

The results of investigation of the cytocidal effect of each construct prepared using the suicide gene *DT-A* are shown in Fig. 6 (colony formation method), Fig. 7 (growth inhibition test), and Fig. 8 (growth inhibition test). It was found that the recombination efficiency decreases when the homology is decreased. The substrate in which the homology between the homologous regions was decreased by 5% (pCMV-SA-DTA95) was found to show the largest difference in the cytocidal effect, that is, the recombination efficiency, between the *Msh2-* cells and the *Msh2+* cells, and to be most sensitive to the presence or absence of mismatch repair. Use of such a substrate can increase the possibility that only cells deficient in mismatch repair can be effectively killed.

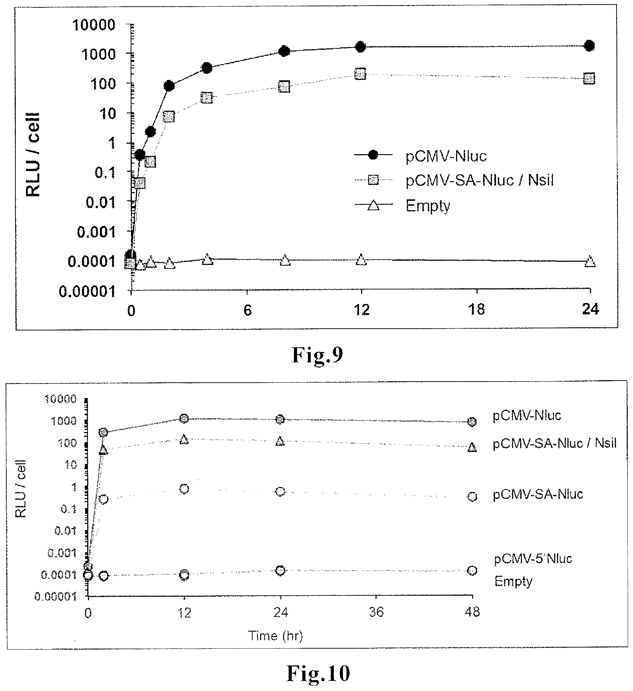

Figs. 9 and 10 show examples of the result of an experiment using the SA-Nluc construct. Within 2 hours of (30 minutes after) the transfection, the luciferase activity (= recombination frequency between the homologous regions) could be detected. It was thus found that the recombination reaction quickly occurs in the outside of the chromosomes after the introduction of the vector, and that quantitative measurement of transient expression of the normal-type gene generated by this recombination can be carried out simply and rapidly with high sensitivity. Further, it was confirmed that the recombination reaction occurs to enable detection of the luciferase activity also in a case where the cells are transfected with a circular construct that has not been linearized by NsiI (pCMV-SA-Nluc in Fig. 10) although the efficiency decreased.

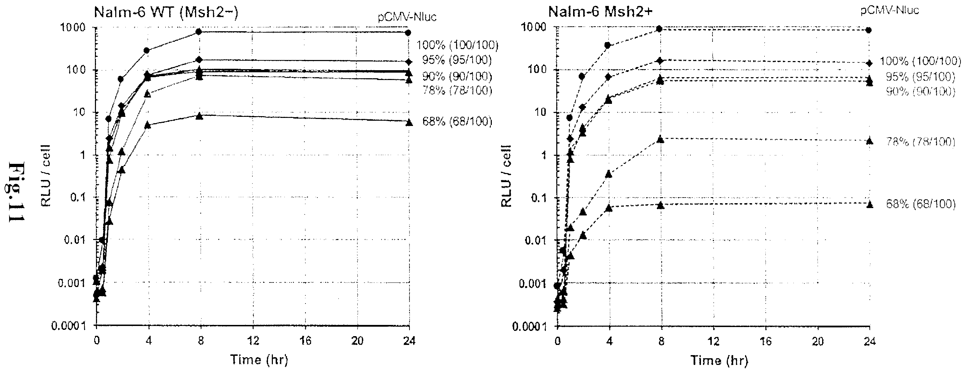

Fig. 11 shows an example of the result of an experiment using SA-Nluc constructs having varied homologies between the homologous regions. It was found that the recombination efficiency decreases as the homology to the luciferase gene *Nluc* is decreased. This tendency was more remarkable in the *Msh2+* cells than in the *Msh2-* cells. In particular, the substrate in which the homology between the homologous regions was decreased by 32% (pCMV-SA-Nluc68) was found to show an about 100-fold difference in the luciferase activity, that is, in the recombination efficiency, between the *Msh2-* cells and the *Msh2*+ cells. It was shown that use of such a substrate enables effective detection of cells deficient in mismatch repair.

The influence of the difference in the size of the homologous region in the SA-DTA construct on the cytocidal effect was investigated. Using MaxCyte (OC-25×3, second time), 2×10⁶ Nalm-6 cells (WT) were transfected with each construct (2 µg). Twenty-four hours after the gene transfer, 200 or 5000 cells were plated on a 60-mm dish, and colonies were allowed to form. The PE after the introduction of each construct was calculated as a relative value to the PE after the introduction of the positive control pCMV-Nluc, which was taken as 1.

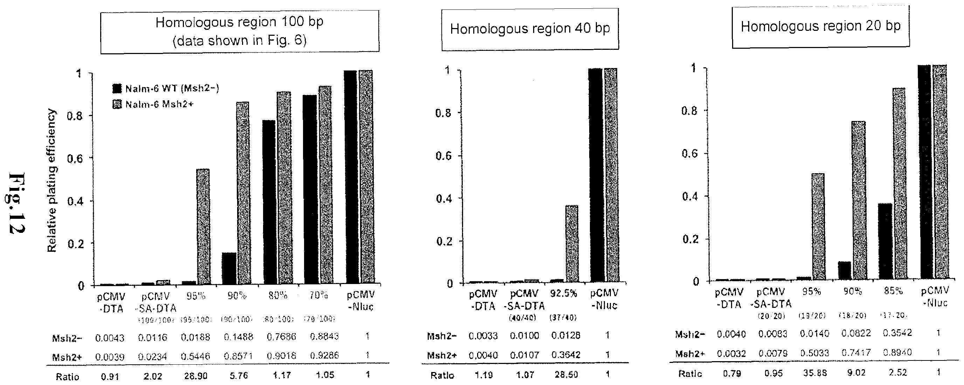

The result of investigation of the cytocidal effect of each construct having shortened homologous regions in the suicide gene *DT-A* is shown in Fig. 12. It was found that, similarly to the case where the length of the homologous region is 100 bp, the recombination efficiency decreases when the length of the homologous region is 40 bp or 20 bp. In the case where the length of the homologous region is 20 bp, the substrate in which the homology between the homologous regions was decreased by 5% (pCMV-SA-DTA20-95) was found to show the largest difference in the cytocidal effect, that is, in the recombination efficiency, between the *Msh2-* cells and the *Msh2+* cells, and to be most sensitive to the presence or absence of mismatch repair. Use of such a substrate can increase the possibility that only cells deficient in mismatch repair can be effectively killed.

### B. Experiment 2

### B-1. Method of Preparation of Vectors (DNA Constructs)

### B-1-1. Construction of pCMV-SA-Nluc-spacer Construct

For preparation of a vector in which a spacer is inserted between the homologous sequences, a spacer fragment (3952 bp; SEQ ID NO:52; positions 1985 to 2002 correspond to an I-Scel recognition sequence) was amplified by PCR using pJB1(Gravells et al. (2015) Use of the HPRT gene to study nuclease-induced DNA double-strand break repair. Hum. Mol. Genet.24: 7097-7110) as a template. As a polymerase for the PCR, PrimeSTAR HS (trade name) DNA Polymerase (Takara Bio Inc.) was used. As primers, Nluc-spacer-Fw (SEQ ID NO:44) and Nluc-spacer-Rv (SEQ ID NO:45) were used. Subsequently, pCMV-SA-Nluc (100% homology), which was prepared in A-1-1-3; and pCMV-SA-Nluc95 (95% homology), pCMV-SA-Nluc90 (90% homology), pCMV-SA-Nluc78 (78% homology), and pCMV-SA-Nluc68 (68% homology), which were prepared in A-1-1-4; were digested with NsiI, and the spacer fragment was inserted between the *5'Nluc* fragment and each 3'*Nluc* fragment having mutations using an In-Fusion HD Cloning Kit (Takara Bio Inc.), to obtain pCMV-SA-Nluc-spacer (100%), pCMV-SA-Nluc95-spacer (95% homology), pCMV-SA-Nluc90-spacer (90% homology), pCMV-SA-Nluc78-spacer (78% homology), and pCMV-SA-Nluc68-spacer (68% homology), in each of which [CMV promoter] - *[5'Nluc* fragment] - [spacer] - [each *3'Nluc* fragment having mutations] - [poly-A addition signal] were linked together. The prepared plasmid was purified using a Qiagen Plasmid Plus Midi Kit (Qiagen K. K.), and linearized by cleavage with I-SceI (New England Biolabs) before using the plasmid in transfection.

### B-1-2. Construction of Divided-Type pCMV-SA-Nluc Construct

A fragment of the *Nluc* gene to be used for pCMV-5'Nluc (a 345-bp 5'*-Nluc* fragment; SEQ ID NO:23) was obtained by PCR amplification using the pNL1.1 [Nluc] Vector (Promega, Madison, WI, USA) as a template. As a polymerase for the PCR, PrimeSTAR HS (trade name) DNA Polymerase (Takara Bio Inc.) was used. As primers, Nluc-Fw (SEQ ID NO:5) and SA-5'Nluc-Rv2 (SEQ ID NO:46) were used. Subsequently, pCMV-Nluc (Saito S, et al. Nature Commun. 8:16112, 2017) was digested with NheI and BamHI, and a fragment containing the CMV promoter was recovered. Using an In-Fusion HD Cloning Kit (Takara Bio Inc.), the *5'Nluc* fragment was inserted downstream of the CMV promoter to obtain pCMV-5'Nluc, in which [CMV promoter] - *[5'Nluc* fragment] were linked together. The prepared plasmid was purified using a Qiagen Plasmid Plus Midi Kit (Qiagen K. K.), and linearized by cleavage with Nsil (Takara Bio Inc.) (cleavage at an NsiI site introduced in the 3'-end portion of the 5'*Nluc* fragment) before using the plasmid in transfection.

A fragment in which the 3'*Nluc* fragment and the poly-A addition signal were linked together (582 bp; SEQ ID NO:53; in this base sequence, positions 21 to 290 correspond to the *Nluc* sequence, and positions 339 to 460 correspond to the poly-A addition signal) was obtained by PCR amplification using pCMV-SA-Nluc, which was prepared in A-1-1-3, as a template. As a polymerase for the PCR, Tks Gflex (trade name) DNA Polymerase (Takara Bio Inc.) was used. As primers, SSA-3'Nluc-Fw (SEQ ID NO:7) and SV40-pA-Rv (SEQ ID NO:47) were used. The amplified DNA fragment was purified using the Wizard SV Gel and PCR Clean-Up System (Promega) and then used in transfection.

### B-1-3. Construction of Divided-Type pCMV-SA-DTA Construct

A *DT-A* gene fragment to be used for pCMV-5'DTA (a 132-bp 5'DTA fragment; SEQ ID NO:11) was obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. As primers, DTA-Fw (SEQ ID NO:1) and SA-5'DTA-Rv2 (SEQ ID NO:48) were used. Subsequently, pCMV-Nluc was digested with NheI and ClaI, and a fragment containing the CMV promoter was recovered. Using an In-Fusion HD Cloning Kit (Takara Bio Inc.), the 5'DTA fragment was inserted downstream of the CMV promoter to obtain pCMV-5'DTA, in which [CMV promoter] - [5'DTA fragment] were linked together. The prepared plasmid was purified using a Qiagen Plasmid Plus Midi Kit (Qiagen K. K.), and linearized by cleavage with I-SceI (New England Biolabs) before using the plasmid in transfection.

A fragment in which the 3'DTA fragment and the poly-A addition signal were linked together (866 bp; SEQ ID NO:54; in this base sequence, positions 25 to 582 correspond to the DTA sequence, and positions 623 to 744 correspond to the poly-A addition signal) was obtained by PCR amplification using pCMV-SA-DTA, which was prepared in A-1-1-1, as a template. As a polymerase for the PCR, Tks Gflex (trade name) DNA Polymerase (Takara Bio Inc.) was used. As primers, SA-3'DTA-Fw2 (SEQ ID NO:49) and SV40-pA-Rv (SEQ ID NO:47) were used. The amplified DNA fragment was purified using the Wizard SV Gel and PCR Clean-Up System (Promega) and then used in transfection.

### B-1-4. Construction ofpCMV-SA-TK Construct

An integrated-type construct using the *HSV-TK* gene as a suicide gene was constructed.

First, for preparing a vector for expression of the *HSV-TK* gene (pCMV-TK), the ORF of the *HSV-TK* gene was amplified by PCR using an *HSV-TK* gene fragment (GenBank: V00470.1, Kobayashi et al. (2001) Decreased topoisomerase IIalpha expression confers increased resistance to ICRF-193 as well as VP-16 in mouse embryonic stem cells. Cancer Lett. 166(1): 71-77; the base sequence of the coding region of the *HSV-TK* gene and the amino acid sequence of the TK protein encoded are shown in SEQ ID NOs:55 and 56) as a template. As a polymerase for the PCR, PrimeSTAR HS (trade name) DNA Polymerase (Takara Bio Inc.) was used. As primers, TK-Fw (SEQ ID NO:66) and TK-Rv (SEQ ID NO:67) were used. Subsequently, pIRES (Takara Bio Inc., Fig. 2) was digested with Nhel and XbaI, and a fragment containing the CMV promoter and the poly-A addition signal was recovered. Using an In-Fusion HD Cloning Kit (Takara Bio Inc.), the *TK* gene fragment was inserted downstream of the CMV promoter to obtain pCMV-TK, in which [CMV promoter] - [*HSV-TK* gene fragment] - [poly-A addition signal] were linked together. The prepared plasmid was purified using a Qiagen Plasmid Plus Midi Kit (Qiagen K. K.) and then used in transfection.

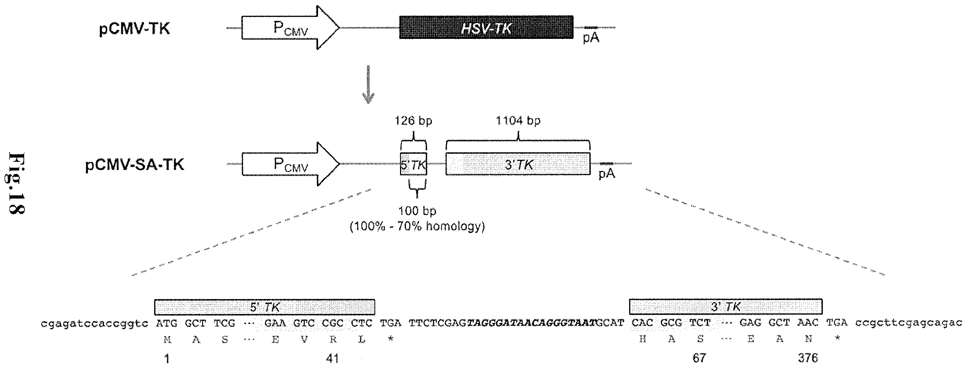

A fragment containing part of 5'*TK* to be used for pCMV-SA-TK (a fragment containing the region corresponding to positions 27 to 124 in the base sequence of the *HSV-TK* gene) was obtained by PCR amplification using an *HSV-TK* gene fragment (GenBank: V00470.1) as a template. As a polymerase for the PCR, PrimeSTAR HS (trade name) DNA Polymerase (Takara Bio Inc.) was used. As primers, SA-TK-Fw (SEQ ID NO:50) and SA-TK-Rv (SEQ ID NO:51) were used. Subsequently, the fragment containing part of 5'*TK* amplified by the PCR was digested with MluI, and inserted into an MluI site of pCMV-TK (present in the *HSV-TK* gene ORF) using a DNA Ligation Kit <Mighty Mix> (Takara Bio Inc.). By this, pCMV-SA-TK, in which [CMV promoter] - [5'*TK* fragment] - [3'*TK* fragment] - [poly-A addition signal] were linked together, was obtained. This construct has an I-SceI recognition site between [5'TK fragment] and [3'TK fragment] (sequence: TGA TTCTCGAGTAGGGATAACAGGGTAATGCAT; SEQ ID NO:61) as shown in Fig. 18. The prepared plasmid was purified using a Qiagen Plasmid Plus Midi Kit (Qiagen K. K.), and linearized by cleavage with I-Scel (New England Biolabs) before using the plasmid in transfection.

**[Table 2]**

| Primer name | Sequence (5'->3') | SEQ ID NO |
|---|---|---|
| Nluc-Spacer-Fw | GACGGCCGTGATTCTAGAGGTACCGGTTGACAGACGTTAGGTTG | 44 |
| Nluc-Spacer-Rv | GATCATCCACAGGGTATGCATAACCAACAGTGAAACAGCG | 45 |
| SA-5'Nluc-Rv2 | CACCATACGCGGATCATGCATCACGGCCGTCCGAAATAGTCGATC | 46 |
| SV40-pA-Rv | GAGTGCACCATACGCGGATC | 47 |
| SA-5'DTA-Rv2 | CGCGGATCCTTATCGATGCATTACCCTGTTATCCCTATCATTGTGTACCAGATT TTGGC | 48 |
| SA-3' DTA-Fw2 | TGATAGGGATAACAGGGTAATGC | 49 |
| SA-TK-Fw | ACACGCGTCTGCGTTCGACC | 50 |
| SA-TK-Rv | ATACGCGTGATGCATTACCCTGTTATCCCTACTCGAGAATCAGAGGCGGACTTC CGTGGCTTCTTG | 51 |
| TK-Fw | TAATACGACTCACTATAGGCTAGCCTCGAGATCCACCGGTCATGGCTTCGTACC CCGGCCATC | 66 |
| TK-Rv | CCGCCCCGACTCTAGAGTCGCGGTCAGTTAGCCTCCCCCATGTCC | 67 |

| | | |
|---|---|---|
| Single underline indicates a I-Scel recognition site. Wavy underline indicates a Nsil recognition site. | | |

### B-2. Cells and Gene Transfer

The human pre-B cell line Nalm-6 (cancer cells deficient in the mismatch repair mechanism) and cells derived therefrom were cultured at 37°C in a 5% CO₂ incubator (So et al. (2004) Genetic interactions between BLM and DNA ligase IV in human cells. J. Biol. Chem. 279: 55433-55442). Transfection of the Nalm-6 cells was carried out by the electroporation method according to a past report (Saito et al. (2017) Dual loss of human POLQ and LIG4 abolishes random integration. Nat. Commun. 8: 16112).

The human colorectal-derived cancer cell line HCT116 (Horizon Discovery, Cambridge, UK) and *MLH1* revertant cells thereof (HCT116 +*MLH1*) (Horizon Discovery) were cultured at 37°C in a 5% CO₂ incubator. As a medium, Dulbecco's modified Eagle medium (Nissui Pharmaceutical Co., Ltd., Tokyo, Japan) supplemented with calf serum (Global Life Science Technologies Japan, Tokyo, Japan; addition of 50 ml calf serum per 500 ml of Eagle MEM) and L(+)-glutamine (Fujifilm Wako Pure Chemical Corporation, Osaka, Japan; addition to a final concentration of 2 mM) was used. Transfection was carried out using jetPEI (Polyplus-transfection, Illkirch, France) according to a protocol provided by the manufacturer.

### B-3. Evaluation of Cytocidal Effect

After transfecting 2×10⁶ Nalm-6 cells with each construct (1 µg), the cytocidal effect was investigated by either the growth inhibition test method or the colony formation method.

### (1) Growth Inhibition Test

The cells after the gene transfer were plated on a 24-well dish at 1×10⁵ cells/ml, and, 24 hours later, ganciclovir (Fujifilm Wako Pure Chemical Corporation) was added thereto to a final concentration of 500 nM. The survival rate of the cells was measured at 48-hour intervals using CellTiter-Glo (CellTiter-Glo (registered trademark) Luminescent Cell Viability Assay, Promega).

### (3) Colony Formation Method

Twenty-four hours after the gene transfer, 200 or 5000 cells were plated on a 60-mm dish, and colonies were allowed to form. When pCMV-SA-TK was transferred, 200 or 5000 cells after the gene transfer were plated on an agar medium supplemented with 500 nM ganciclovir. After culturing the cells for 17 days, the grown colonies were counted, and the survival rate was calculated.

### B-4. Luciferase Assay

A luciferase assay of Nalm-6 cells and their derived cell lines was carried out as follows. With each construct (in an amount of 2 µg, 1 µg, 0.2 µg, 0.02 µg, or 0.002 µg), 2×10⁶ Nalm-6 cells were transfected. The cells after the gene transfer were plated on a 24-well dish at 5×10⁵ cells/ml, and the luciferase activity (RLU value) was measured immediately after the gene transfer (0 hour later), 1 hour later, 2 hours later, 4 hours later, 8 hours later, and 24 hours later using the Nano-Glo Luciferase Assay System (Promega).

A luciferase assay of HCT116 cells and *MLH1* revertant cells thereof (HCT116 +*MLH1*) was carried out as follows. On a 24-well dish, 5×10⁴ cells were plated and cultured overnight, followed by transfecting the cells with each construct (1 µg). Twenty-four hours after the gene transfer, the cell number was counted, and the luciferase activity (RLU value) was measured using the Nano-Glo Luciferase Assay System (Promega).

### B-5. Results

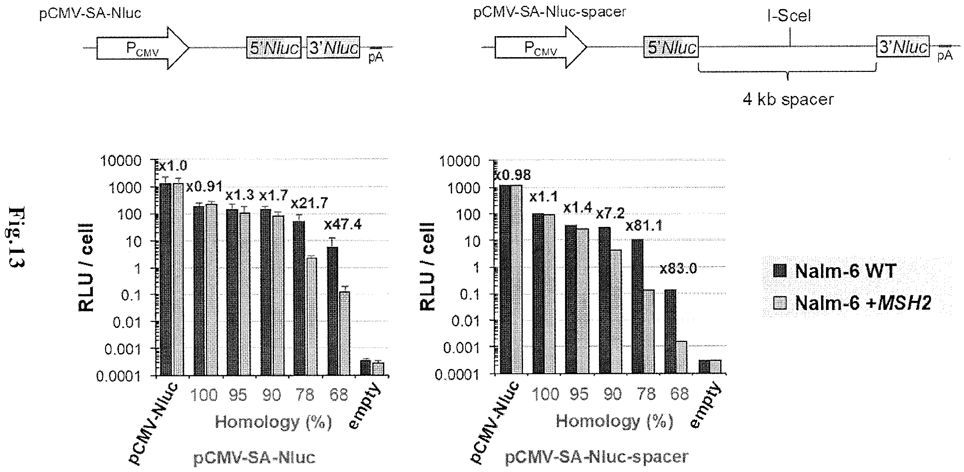

As one example of the result of an experiment using pCMV-SA-Nluc-spacer constructs, Fig. 13 shows the result of a luciferase assay of Nalm-6 cells and *MSH2* revertant cells thereof (Nalm-6 +*MSH2*)*.* Compared to the pCMV-SA-Nluc construct in which no spacer was inserted (Fig. 13, left), CMV-SA-Nluc-spacer constructs in which a long spacer was inserted tended to more clearly show the difference in the recombination efficiency between the mismatch repair-deficient cells and proficient cells.

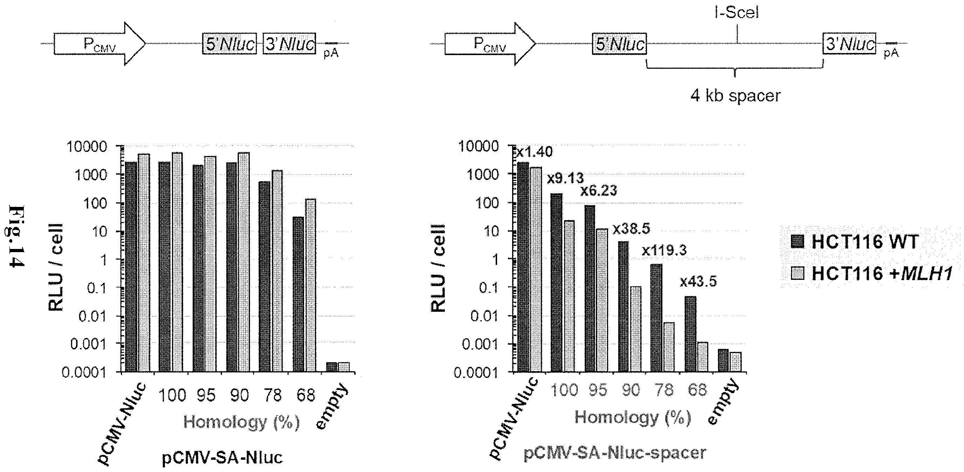

As another example of the result of an experiment using pCMV-SA-Nluc-spacer constructs, Fig. 14 shows the result of a luciferase assay of HCT116 cells and *MLH1* revertant cells thereof (HCT1 16 +*MLH1*). It could be confirmed that, not only in the case of mismatch repair deficiency caused by *MSH2,* but also in the case of mismatch repair deficiency caused by *MLH1,* the difference in gene expression due to the presence or absence of the deficiency increases when the homology is decreased. This result suggests that the construct of the present invention is not affected by the cause of mismatch repair deficiency.

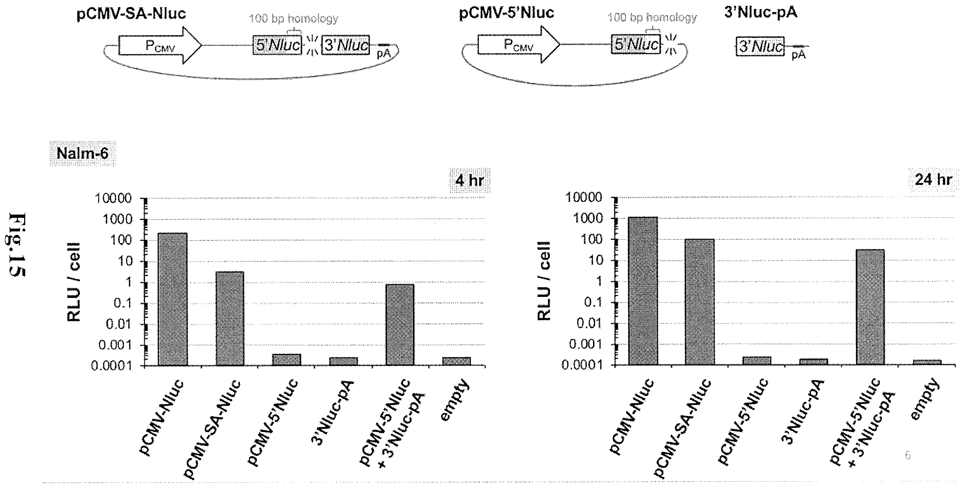

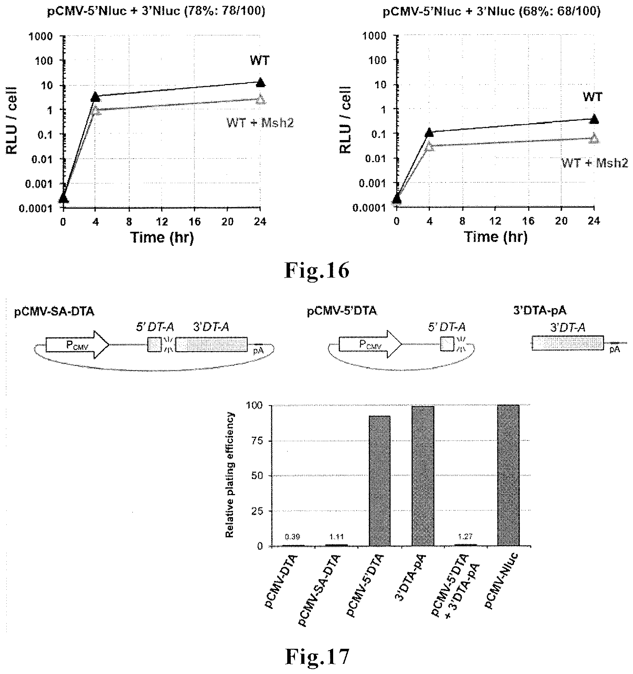

Figs. 15 and 16 show examples of the result of a luciferase assay using a divided-type SA-Nluc construct. The divided-type construct (pCMV-5'Nluc + 3'Nluc-pA) showed almost the same level of effectiveness as that of the integrated type (pCMV-SA-Nluc) (Fig. 15). Further, it was found that the divided-type construct also shows a clearer difference in the gene expression when the homology was decreased (Fig. 16).

Fig. 17 shows an example of the result of evaluation of the cytocidal effect in Nalm-6 cells transfected with a divided-type SA-DTA construct by the colony formation method. It was confirmed that almost the same level of cytocidal effect as that of the integrated type can be obtained also when the 5' fragment and the 3' fragment of SA-DTA are transfected in a state where these fragments are divided from each other.

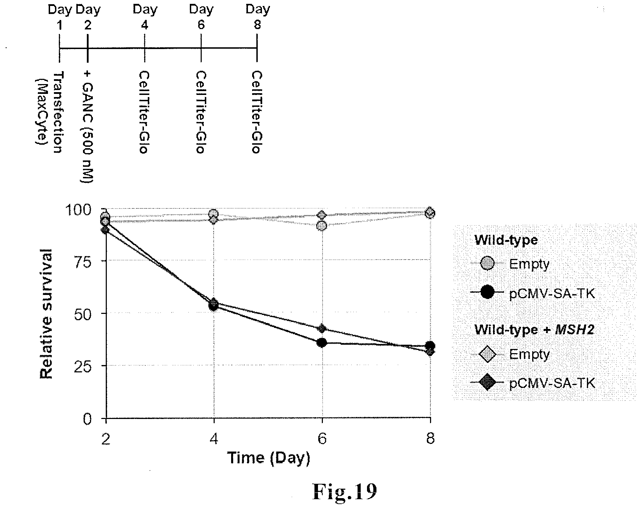

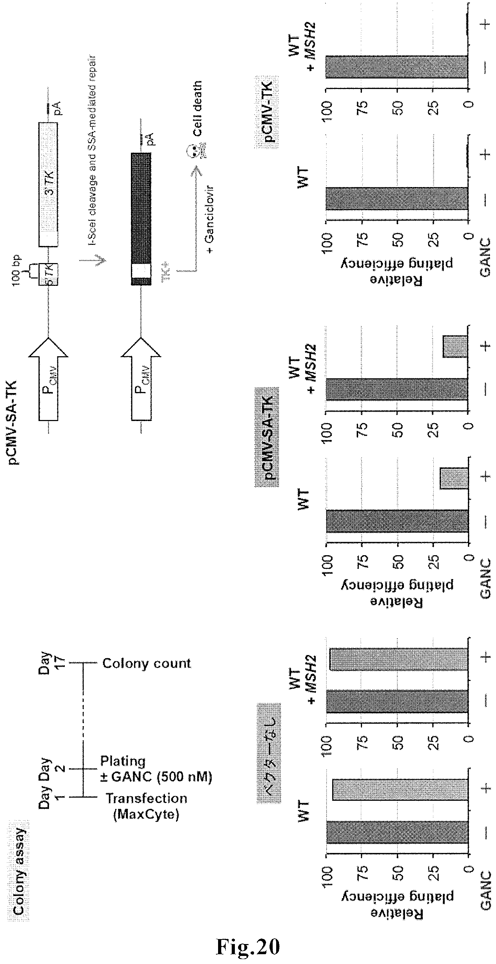

Fig. 18 shows the structure of the integrated-type pCMV-SA-TK construct. One example of the result of evaluation of the cytocidal effect using this construct and Nalm-6 cells is shown in Fig. 19 (growth inhibition test; the survival rate of the cells 6 days after the gene transfer is expressed as a relative value to the value obtained without addition of ganciclovir (GANC), which is taken as 100) and Fig. 20 (colony formation method). It was confirmed that, also by using *HSV-TK* as the suicide gene, an effect similar to that obtained with the construct using *DT-A* is obtained.

### C. Experiment 3

### C-1. Construction of pCMV-SA-SecNluc Construct

For preparation of a vector that expresses an *Nluc* gene to which a secretory signal is added, a secretory signal sequence derived from human interleukin 6 (IL6) was prepared by artificial gene synthesis (pIL6-Nluc). The pIL6-Nluc prepared by the artificial gene synthesis was digested with SmaI and EcoNI, and a fragment containing the secretory signal sequence was recovered. The recovered fragment containing the secretory signal was mixed with a DNA fragment obtained by digesting pCMV-Nluc with SmaI and EcoNI, and the fragment containing the secretory signal sequence was inserted upstream of the *Nluc* gene using a DNA Ligation Kit <Mighty Mix> (Takara Bio Inc.), to obtain pCMV-SecNluc, in which [CMV promoter] - [secretory signal] - [*Nluc* gene] - [poly-A addition signal] were linked together. The base sequence of the [secretory signal] - [*Nluc* gene] portion (*SecNluc* gene) and the amino acid sequence encoded thereby are shown in SEQ ID NOs:62 and 63. Positions 1 to 87 of the *SecNluc* gene sequence of SEQ ID NO:62 correspond to the DNA sequence encoding the secretory signal, and positions 1 to 29 of the amino acid sequence of SEQ ID NO:63 correspond to the amino acid sequence of the secretory signal.

For adding the secretory signal sequence to the 5'*Nluc* gene of pCMV-SA-Nluc (100% homology) and the 5'*Nluc* gene of pCMV-SA-Nluc68 (68% homology), pCMV-SecNluc was digested with XhoI and EcoNI, and a fragment containing the secretory signal sequence was recovered. Subsequently, each of pCMV-SA-Nluc (100% homology) and pCMV-SA-Nluc68 (68% homology) was digested with XhoI and EcoNI, and a fragment containing the CMV promoter was recovered. Finally, the recovered fragment containing the secretory signal sequence was mixed with the fragment containing the CMV promoter, and the fragment containing the secretory signal was inserted upstream of the 5'*Nluc* gene using a DNA Ligation Kit <Mighty Mix> (Takara Bio Inc.), to obtain pCMV-SA-SecNluc (100% homology), in which [CMV promoter] - [secretory signal] - [5'*Nluc* fragment] - [3'*Nluc* fragment] - [poly-A addition signal] were linked together, and pCMV-SA-SecNluc68 (68% homology), in which [CMV promoter] - [secretory signal] - [5'*Nluc* fragment] - [3'*Nluc* fragment having mutations] - [poly-A addition signal] were linked together. The base sequence of the [secretory signal] - [5'*Nluc* fragment] portion (5'*SecNluc* fragment) and the amino acid sequence encoded thereby are shown in SEQ ID NOs:64 and 65.

### C-2. Cells, Gene Transfer, and Luciferase Assay

### C-2-1. Cells and Gene Transfer

The human pre-B cell line Nalm-6 and cells derived therefrom were cultured at 37°C in a 5% CO₂ incubator (So et al. (2004) Genetic interactions between BLM and DNA ligase IV in human cells. J. Biol. Chem. 279: 55433-55442). Transfection of the Nalm-6 cells was carried out by the electroporation method according to a past report (Saito et al. (2017) Dual loss of human POLQ and LIG4 abolishes random integration. Nat. Commun. 8: 16112).

The human colorectal-derived cancer cell line HCT116 (Horizon Discovery, Cambridge, UK) and *MLH1* revertant cells thereof (HCT116 +*MLH1*) were cultured at 37°C in a 5% CO₂ incubator. As a medium, Dulbecco's modified Eagle medium (Nissui Pharmaceutical Co., Ltd., Tokyo, Japan) supplemented with calf serum (Global Life Science Technologies Japan, Tokyo, Japan; addition of 50 ml calf serum per 500 ml of Eagle MEM) and L(+)-glutamine (Fujifilm Wako Pure Chemical Corporation, Osaka, Japan; addition to a final concentration of 2 mM) was used. Transfection was carried out using jetPEI (Polyplus-transfection, Illkirch, France) according to a protocol provided by the manufacturer.

### C-2-2. Luciferase Assay

A luciferase assay of Nalm-6 cells and their derived cell lines was carried out as follows. With each construct (2 µg), 2×10⁶ Nalm-6 cells were transfected. The cells after the gene transfer were plated on a 24-well dish at 5×10⁵ cells/ml, and the luciferase activity (RLU value) was measured at Hour 2 and Hour 24 after the gene transfer using the supernatant (50 µl) of the cell culture, using the Nano-Glo Luciferase Assay System (Promega).

### C-3. Results

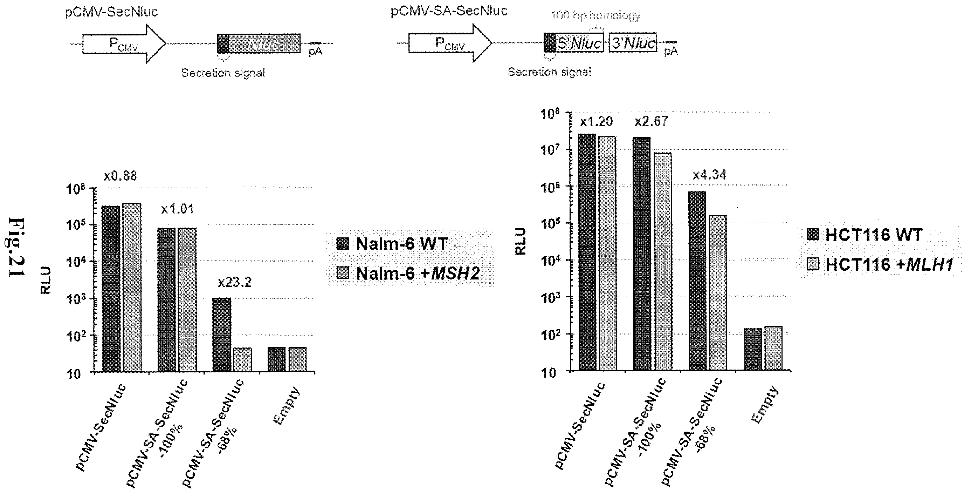

The result of the measurement of the luciferase activity using the culture supernatant is shown in Fig. 21. The result obtained was similar to that obtained with a non-secretory luciferase construct. When using a secretory luciferase, the cell lysis treatment is not necessary, and thus the assay can be carried out using a culture supernatant in a simple manner.

### D. Experiment 4

### D-1. Preparation of Vector

Two *Nluc* gene fragments (a 5*'Nluc*-40 fragment (SEQ ID NO:74), which has a length of 121 bp, and a 3*'Nluc*-40 fragment (SEQ ID NO:79), which has a length of 432 bp) to be used for pCMV-SA-Nluc-40 (100% homology) having a homologous region with a length of 40 bp were obtained by PCR amplification using the pNL1.1 [Nluc] Vector (Promega, Madison, Wl, USA) as a template. As a polymerase for the PCR, PrimeSTAR HS (trade name) DNA Polymerase (Takara Bio Inc.) was used. The primers used are shown below in Table 3. Nluc-Fw (SEQ ID NO:5) and SA-5'Nluc-40-Rv (SEQ ID NO:68) were used for the amplification of the 5'*Nlue* fragment, and SA-3'Nluc-40-Fw (SEQ ID NO:69) and Nluc-Rv (SEQ ID NO:8) were used for the amplification of the 3*'Nluc* fragment. Subsequently, pIRES (Takara Bio Inc., Fig. 2) was digested with NheI and XbaI, and a fragment containing the CMV promoter and the poly-A addition signal was recovered. Using an In-Fusion HD Cloning Kit (Takara Bio Inc.), the 5'*Nluc-*40 fragment and the *3'Nluc-40* fragment were inserted downstream of the CMV promoter, to obtain pCMV-SA-Nluc-40 (100% homology), in which [CMV promoter] - [5'*Nluc-*40 fragment] - [3'*Nluc*-40 fragment] - [poly-A addition signal] were linked together. The prepared plasmid was purified using a Qiagen Plasmid Plus Midi Kit (Qiagen K. K.), and linearized by cleavage between [5'*Nluc*-40 fragment] and [3'*Nluc*-40 fragment] with NsiI (Takara Bio Inc.) before using the plasmid in transfection.

Subsequently, for preparation of a vector in which the homology between the homologous sequences was decreased, a DNA fragment was amplified by PCR by introducing silent mutations into the DNA sequence homologous to 5'*Nluc*-40 (40 bp, the region corresponding to positions 1 to 40 in SEQ ID NO:79) in the 3'*Nluc*-40 sequence. The silent mutations were introduced such that the homology to 5'*-Nluc-*40 should be 90% (4 sites), 70% (12 sites), 60% (16 sites), or 50% (20 sites) (however, for the DNA sequences encoding the 33rd amino acid (phenylalanine) and the 37th amino acid (glycine) of the *Nluc* gene, mutations that substitute each amino acid with leucine or glycine were used as well as silent mutations; these two amino acid substitutions have been known not to affect the luciferase activity of Nluc protein). As a polymerase for the PCR, PrimeSTAR HS (trade name) DNA Polymerase (Takara Bio Inc.) was used. The primers used are shown below in Table 3. SA-3'Nluc-40-90%-Fw (SEQ ID NO:70) and Nluc-Rv (described above) were used for the amplification of 3'*Nluc*-40 (90% homology) (SEQ ID NO:80); SA-3'Nluc-40-70%-Fw (SEQ ID NO:71) and Nluc-Rv (SEQ ID NO:8) were used for the amplification of 3*'Nluc*-40 (70% homology) (SEQ ID NO:81); SA-3'Nluc-40-60%-Fw (SEQ ID NO:72) and Nluc-Rv (SEQ ID NO:8) were used for the amplification of *3'Nluc-40* (60% homology) (SEQ ID NO:82); and SA-3'Nluc-40-50%-Fw (SEQ ID NO:73) and Nluc-Rv (SEQ ID NO:8) were used for the amplification of 3'*Nluc*-40 (50% homology) (SEQ ID NO:83). Subsequently, pIRES (Takara Bio Inc.) was digested with NheI and XbaI, and a fragment containing the CMV promoter and the poly-A addition signal was recovered. Using an In-Fusion HD Cloning Kit (Takara Bio Inc.), the 5'*Nluc*-40 fragment and each 3'*Nluc*-40 fragment having mutations were inserted downstream of the CMV promoter, to obtain pCMV-SA-Nluc-40 (90% homology), pCMV-SA-Nluc-40 (70% homology), pCMV-SA-Nluc-40 (60% homology), and pCMV-SA-Nluc-40 (50% homology), in each of which [CMV promoter] - [5*'Nluc*-40 fragment] - [each *3'Nluc-40* fragment having mutations] - [poly-A addition signal] were linked together. The prepared plasmid was purified using a Qiagen Plasmid Plus Midi Kit (Qiagen K. K.), and linearized by cleavage between [5'*Nluc*-40 fragment] and [3'*Nluc*-40 fragment] with NsiI (Takara Bio Inc.) before using the plasmid in transfection.

**[Table 3]**

| Primer name | Sequence (5'→3') | SEQ ID NO |
|---|---|---|
| Nluc-Fw | TAATACGACTCACTATAGGCTAG | 5 |
| SA-5'Nluc-40-Rv | ATGCATTACCCTGTTATCCCTATCATATTACGGACACCCCGAGATTCTGA | 68 |
| SA-3'Nluc-40-Fw | GGGATAACAGGGTAATGCATCGGTGTGTCCAGTTTGTTTCAGAATCTCG | 69 |
| Nluc-Rv | CCGCCCCGACTCTAGAGTCGCGG | 8 |
| SA-3'Nluc-40-90%-Fw | GGGATAACAGGGTAATGCATCGGTGTGTCTAGTTTGTTCCAGAATCTGGGG GTGTCTGTAAC | 70 |
| SA-3'Nluc-40-70%-Fw | GGGATAACAGGGTAATGCATCGGAGTCTCTAGCTTATTCCAAAACCTGGGC GTCTCTGTAAC | 71 |
| SA-3' N i u c-40-60%-Fw | GGGATAACAGGGTAATGCATCGGAGTCAGCAGCTTACTCCAAAACCTGGGC GTCAGCGTGACTCC | 72 |
| SA-3'Nluc-40-50%-Fw | GGGATAACAGGGTAATGCATCGGAGTCAGCTCTCTCCTCCAAAACTTGGCC GTCAGCGTGACTCC | 73 |

### D-2. Method

A luciferase assay of Nalm-6 cells and their derived cell lines was carried out in the same manner as described above in C-2.

### D-3. Results

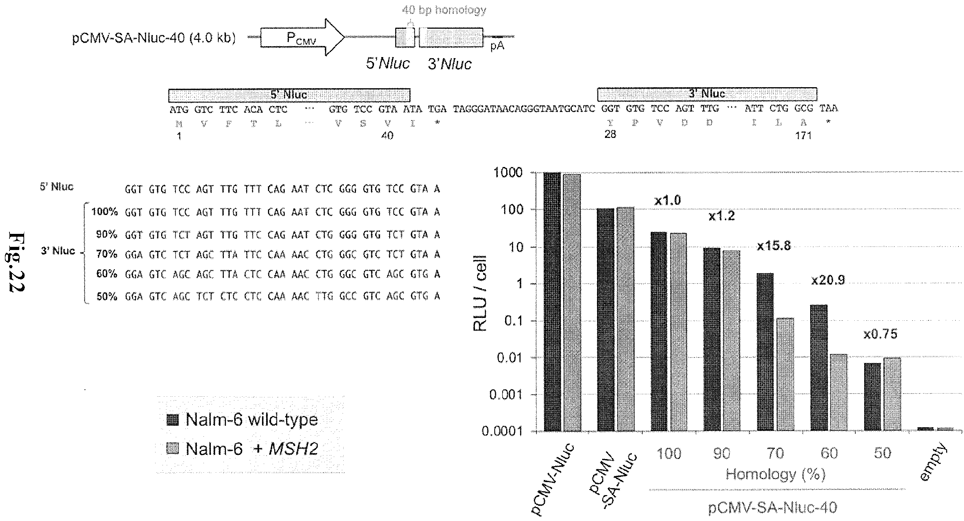

As shown in Fig. 22, as the DNA homology between the homologous regions in the construct decreased, the influence of the *Msh2* deficiency began to appear (the 60%-construct showed the largest difference). It was suggested, however, that no recombination may occur when the homology is decreased to 50%.

## Claims

1. A nucleic acid construct comprising: a promoter region; and a 5'-side region and a 3'-side region of a gene sequence encoding a protein; the 5'-side region having a first homologous region linked downstream thereof, the 3'-side region having a second homologous region linked upstream thereof, the first homologous region and the second homologous region being homologous to each other.

2. The nucleic acid construct according to claim 1, wherein the nucleic acid construct is a circular nucleic acid construct comprising, downstream of the promoter region, the 5'-side region and the 3'-side region, or a linear nucleic acid construct prepared by cleaving the circular nucleic acid construct between the 5'-side region and the 3'-side region; or a linear nucleic acid construct comprising, downstream of the promoter region, the 5'-side region and the 3'-side region.

3. A nucleic acid construct comprising: a promoter region; a 5'-side region and a 3'-side region of a gene sequence encoding a protein; a first homologous region linked downstream of the 5'-side region; and a second homologous region linked upstream of the 3'-side region; the regions being placed in two different nucleic acid molecules, wherein a first nucleic acid molecule comprises, downstream of the promoter region, the 5'-side region and the first homologous region, and a second nucleic acid molecule comprises the 3'-side region and the second homologous region.

4. The nucleic acid construct according to any one of claims 1 to 3, wherein the 5'-side region and the 3'-side region are regions designed by dividing the gene sequence encoding the protein into two parts such that an overlapping region is included in each part, wherein the overlapping region present in the 3'-end portion within the 5'-side region is the first homologous region, and the overlapping region present in the 5'-end portion within the 3'-side region is the second homologous region.

5. The nucleic acid construct according to any one of claims 1 to 3, wherein the first homologous region and the second homologous region are each composed of a non-coding sequence or at least part of a sequence encoding a protein different from the previously-mentioned protein.

6. The nucleic acid construct according to any one of claims 1 to 5, comprising a poly-A addition signal downstream of the 3'-side region.

7. The nucleic acid construct according to any one of claims 1 to 6, wherein the first homologous region and the second homologous region each have a chain length of 4 bases to 10,000 bases.

8. The nucleic acid construct according to any one of claims 1 to 7, wherein the homology between the first homologous region and the second homologous region is 40% to 100%.

9. The nucleic acid construct according to any one of claims 1 to 8, wherein the gene sequence is a gene sequence encoding a protein that acts to decrease cell survival rate, or a protein whose intracellular expression is detectable.

10. The nucleic acid construct according to claim 9, wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

11. A therapeutic agent for mismatch repair-deficient cancer, the agent comprising the nucleic acid construct according to any one of claims 1 to 10, wherein the gene sequence is a gene sequence encoding a protein that acts to decrease cell survival rate.

12. The therapeutic agent according to claim 11, wherein the gene sequence is a sequence of a suicide gene, a DNA damage-inducing gene, or a DNA repair-inhibiting gene.

13. A diagnostic agent for mismatch repair-deficient cancer, the agent comprising the nucleic acid construct according to any one of claims 1 to 10.

14. The diagnostic agent according to claim 13, wherein the gene sequence is a gene sequence encoding a protein whose intracellular expression is detectable.

15. A companion diagnostic agent for predicting an effect of an anticancer drug for mismatch repair-deficient cancer, the agent comprising the nucleic acid construct according to any one of claims 1 to 10.

16. The companion diagnostic agent according to claim 15, wherein the anticancer drug is an immune checkpoint inhibitor.

17. The companion diagnostic agent according to claim 15 or 16, wherein the gene sequence is a gene sequence encoding a protein whose intracellular expression is detectable.

18. A therapeutic method for mismatch repair-deficient cancer, the method comprising administering the nucleic acid construct according to any one of claims 1 to 10 to a patient with the mismatch repair-deficient cancer, wherein the gene sequence is a gene sequence encoding a protein that acts to decrease cell survival rate.

19. A diagnostic method for mismatch repair-deficient cancer, the method comprising:
introducing the nucleic acid construct according to any one of claims 1 to 10 into cancer cells of a cancer patient; and
measuring expression of the protein.

20. The method according to claim 19, wherein the expression of the protein is measured by directly or indirectly measuring the activity of the protein.

21. The method according to claim 19 or 20, wherein the cancer cells are cells separated from the cancer patient, and the introduction of the nucleic acid construct into the cancer cells is carried out in vitro.

22. The method according to claim 19 or 20, wherein:
the protein is a protein whose intracellular expression is detectable as a signal;
the introduction of the nucleic acid construct into the cancer cells is carried out by administering the nucleic acid construct to the cancer patient; and
whether or not the signal of the protein is detected from a cancer lesion is investigated.

23. The method according to claim 19 or 20, wherein:
the protein is a secretory protein whose intracellular expression is detectable;
the introduction of the nucleic acid construct into the cancer cells is carried out by administering the nucleic acid construct to the cancer patient; and
the activity of the protein in blood separated from the patient after the administration of the nucleic acid construct is measured.

24. A method of predicting an effect of an anticancer drug for mismatch repair-deficient cancer, the method comprising:
introducing the nucleic acid construct according to any one of claims 1 to 10 into cancer cells of a cancer patient; and
measuring expression of the protein.

25. The method according to claim 24, wherein the anticancer drug is an immune checkpoint inhibitor.

26. The method according to claim 24 or 25, wherein the cancer cells are cells separated from the patient, and the introduction of the nucleic acid construct into the cancer cells is carried out in vitro.

27. The method according to claim 24 or 25, wherein:
the protein is a protein whose intracellular expression is detectable as a signal;
the introduction of the nucleic acid construct into the cancer cells is carried out by administering the nucleic acid construct to the patient; and
whether or not the signal of the protein is detected from a cancer lesion is investigated.

28. The method according to claim 24 or 25, wherein:
the protein is a secretory protein whose intracellular expression is detectable;
the introduction of the nucleic acid construct into the cancer cells is carried out by administering the nucleic acid construct to the cancer patient; and
the activity of the protein in blood separated from the patient after the administration of the nucleic acid construct is measured.

29. A method of producing the nucleic acid construct according to claim 3, the method comprising:
amplifying the first nucleic acid molecule by PCR using, as a template, an expression vector for the protein, the vector containing: the promoter region; the gene sequence encoding the protein; and a poly-A addition signal; and using set 1 of forward primer 1-1 that is set upstream of the promoter and reverse primer 1-2 that is set in a partial region α in the gene sequence; and
amplifying the second nucleic acid molecule by PCR using the expression vector as a template, and using set 2 of forward primer 2-1 that is set in a partial region β which is another partial region in the gene sequence and is positioned upstream of the partial region α, and reverse primer 2-2 that is set downstream of the poly-A addition signal or between the gene sequence and the poly-A addition signal.
